(19) Europäisches Patentamt
European Patent Office
Office européen des brevets

(11) EP 2 312 308 B1

(12) EUROPEAN PATENT SPECIFICATION

(45) Date of publication and mention
of the grant of the patent:
31.08.2016 Bulletin 2016/35

(51) Int Cl.:
G01N 27/447 (2006.01)      B01D 57/02 (2006.01)
G01N 33/49 (2006.01)       G01N 33/72 (2006.01)
C07K 1/26 (2006.01)

(21) Application number: 09800373.4

(22) Date of filing: 17.07.2009

(86) International application number:
PCT/JP2009/063010

(87) International publication number:
WO 2010/010859 (28.01.2010 Gazette 2010/04)

(54) **APPARATUS AND METHOD FOR ANALYSIS BY CAPILLARY ELECTROPHORETIC METHOD**

VORRICHTUNG UND VERFAHREN ZUR ANALYSE MITTELS
KAPILLARELEKTROPHORESEVERFAHREN

APPAREIL ET PROCÉDÉ D ANALYSE PAR UN PROCÉDÉ ÉLECTROPHORÉTIQUE CAPILLAIRE

(84) Designated Contracting States:
AT BE BG CH CY CZ DE DK EE ES FI FR GB GR
HR HU IE IS IT LI LT LU LV MC MK MT NL NO PL
PT RO SE SI SK SM TR

(30) Priority: 22.07.2008 JP 2008188840

(43) Date of publication of application:
20.04.2011 Bulletin 2011/16

(73) Proprietors:
• ARKRAY, Inc.
Minami-ku
Kyoto-shi
Kyoto 601-8045 (JP)
• National Institute of Advanced Industrial Science
and Technology
Tokyo 100-8921 (JP)

(72) Inventors:
• SUGIYAMA, Koji
Kyoto-shi,
Kyoto 601-8045 (JP)
• HIGASHIISOKAWA, Yukio
Kyoto-shi,
Kyoto 615-0937 (JP)
• NAKAYAMA, Yusuke
Kyoto-shi,
Kyoto 601-8045 (JP)
• TANAKA, Yoshihide
Osaka 563-8577 (JP)

(74) Representative: Piésold, Alexander James
Dehns
St Bride's House
10 Salisbury Square
London EC4Y 8JD (GB)

(56) References cited:
WO-A1-97/12995          WO-A1-2005/103671
WO-A1-2008/029684       WO-A1-2008/078781
WO-A1-2008/139867       WO-A2-03/102539
JP-A- 2005 265 675      JP-T- 2005 519 669
US-A- 5 431 793         US-A1- 2002 092 770
US-A1- 2003 205 469

• MIYADO T ET AL: "Simultaneous determination
of nitrate and nitrite in biological fluids by
capillary electrophoresis and preliminary study
on their determination by microchip capillary
electrophoresis", JOURNAL OF
CHROMATOGRAPHY, ELSEVIER SCIENCE
PUBLISHERS B.V, NL, vol. 1051, no. 1-2, 8
October 2004 (2004-10-08), pages 185-191,
XP004587115, ISSN: 0021-9673, DOI:
10.1016/J.CHROMA.2004.08.037
• FRANTZEN F: "Chromatographic and
electrophoretic methods for modified
hemoglobins", JOURNAL OF
CHROMATOGRAPHY B: BIOMEDICAL
SCIENCES & APPLICATIONS, ELSEVIER,
AMSTERDAM, NL, vol. 699, no. 1-2, 10 October
1997 (1997-10-10), pages 269-286, XP004095000,
ISSN: 1570-0232, DOI:
10.1016/S0378-4347(97)00245-4

## EP 2 312 308 B1

**Description**

<u>Technical Field</u>

The present invention relates to capillary electrophoresis analysis apparatuses and analysis methods for analyzing protein contained in blood by capillary electrophoresis methods.

<u>Background Art</u>

Proteins such as albumin, globulin ($\alpha$1, $\alpha$2, $\beta$, $\Gamma$ globulin), fibrinogen, hemoglobin, and the like are contained in blood. Ratios, mutations, and the like of these proteins are analyzed and used for diagnosis of diseases. Among these proteins, albumin, globulin ($\alpha$1, $\alpha$2, $\beta$, $\Gamma$ globulin), and fibrinogen are contained in blood in large amounts. The ratios of these proteins are considered as important indicators in diagnosis of cirrhosis, nephrotic syndrome, collagen disease, and the like and are analyzed using cellulose acetate membrane electrophoresis or the like, for example. Further, hemoglobin (Hb) includes hemoglobin A (HbA), hemoglobin F (HbF), hemoglobin S (HbS), glycated hemoglobin, and the like. Among them, HbA and HbF are normal hemoglobin in human. HbS is an abnormal hemoglobin, in which 6th glutamic acid of $\beta$ chain is substituted to valine, and is an indicator in the diagnosis of sickle-cell anemia. Since the glycated hemoglobin is hemoglobin that is reacted with glucose in blood and reflects the past history of the blood glucose level in a biological body, it is used as an indicator in diagnosis, treatment, and the like of diabetes. Among glycated hemoglobin, hemoglobin A1c (HbA1c) whose $\beta$ chain N-terminal valine is glycated is an especially important indicator and is an examination item of routine physical examinations. As described above, proteins in blood including hemoglobin are important indicators of various diseases. Hence, the development of analysis apparatuses, which are applicable in laboratory tests or the like, low in running costs, and reduced in size, is desired.

Examples of the method of measuring hemoglobin in blood include immunological methods, enzymatic methods, affinity chromatography methods, HPLC methods, capillary electrophoresis methods, and the like. Since the immunological methods and the enzymatic methods can be applied to autoanalysis apparatuses, they have advantages of handling large numbers of specimens. However, the immunological methods and the enzymatic methods lack measurement accuracy and require about 10 minutes for analysis. With respect to the affinity chromatography methods, in separation principle, a measurement accuracy of HbA1c is low and it requires about two minutes for analysis. The HPLC methods are used widely as methods of measuring hemoglobin (for example, Patent Document 1). However, the HPLC methods require large and expensive special apparatuses, are difficult to reduce size and cost of the apparatuses, and require about 1 minute for analysis. With respect to the capillary electrophoresis methods, the apparatuses can be downsized using microchips and analysis can be performed in about 90 seconds (for example, Patent Document 2). However, from the viewpoint of using in laboratory tests, further reduction of analysis time is required.

[Patent Document 1] JP3429709 B
[Patent Document 2] WO 2008/047703 A1

US 2002/092770, US 5431793, WO 2008/029684 and WO 2008/139867 disclose examples of capillary electrophoresis analysis apparatus and methods.

<u>Disclosure of Invention</u>

Hence, the present invention is intended to provide an analysis apparatus and analysis method, for analyzing by a capillary electrophoresis method, that allows the whole apparatus to be small, an operation to be simple, a running cost to be low, and protein contained in blood to be analyzed highly accurately in a short time.

In order to achieve the aforementioned object, an analysis apparatus of the present invention is defined in claim 5.

The analysis method of the present invention is defined in claim 1.

The capillary electrophoresis analysis apparatus of the present invention allows the whole apparatus to be small, an operation to be simple, a running cost to be low, and protein in blood to be analyzed highly accurately in a short time such as 35 seconds or shorter. Further, the analysis method of the present invention allows an operation to be simple, a running cost to be low, an analysis apparatus capable of downsizing to be used, and protein in blood to be analyzed highly accurately in a short time such as 35 seconds or shorter. Particularly, the capillary electrophoresis analysis apparatus of the present invention and the analysis method using the same is suitable for micro total analysis systems ($\mu$TAS).

Description of the Embodiments

In the capillary electrophoresis analysis apparatus of the present invention, an inner wall surface of the capillary channel for sample analysis may have an ionic functional group, pH of the electrophoresis running buffer may be in a range of 4.0 to 6.0, and an electroosmotic flow generated at the time of voltage application may be 3 cm/min or more.

In the capillary electrophoresis analysis apparatus of the present invention, the electrophoresis running buffer contains sulfated polysaccharide.

The sulfated polysaccharide is a chondroitin sulfate.

With respect to the capillary electrophoresis analysis apparatus of the present invention, any one of the following conditions (A) to (D) is satisfied:

(A) in the capillary channel for sample analysis, in a case where the cross-sectional shape is rectangular having a width and a depth of 30 $\mu$m each, when the distance from the electrophoresis starting point to the detecting point is 0.25 cm or longer and shorter than 0.75 cm, an electric field for separation is 300 V/cm or less,
0.75 cm or longer and shorter than 1.25 cm, an electric field for separation is in a range of 300 to 600 V/cm,
1.25 cm or longer and shorter than 1.75 cm, an electric field for separation is in a range of 400 to 650 V/cm,
1.75 cm or longer and shorter than 2.25 cm, an electric field for separation is in a range of 450 to 700 V/cm,

(B) in the capillary channel for sample analysis, in a case where the cross-sectional shape is rectangular having a width and a depth of 40 $\mu$m each, when the distance from the electrophoresis starting point to the detecting point is 0.25 cm or longer and shorter than 0.75 cm, an electric field for separation is 250 V/cm or less,
0.75 cm or longer and shorter than 1.25 cm, an electric field for separation is in a range of 250 to 500 V/cm,
1.25 cm or longer and shorter than 1.75 cm, an electric field for separation is in a range of 375 to 550 V/cm,
1.75 cm or longer and shorter than 2.25 cm, an electric field for separation is in a range of 400 to 600 V/cm,

(C) in the capillary channel for sample analysis, in a case where the cross-sectional shape is rectangular having a width and a depth of 50 $\mu$m each, when the distance from the electrophoresis starting point to the detecting point is 0.25 cm or longer and shorter than 0.75 cm, an electric field for separation is 200 V/cm or less,
0.75 cm or longer and shorter than 1.25 cm, an electric field for separation is in a range of 200 to 450 V/cm,
1.25 cm or longer and shorter than 1.75 cm, an electric field for separation is in a range of 300 to 500 V/cm,
1.75 cm or longer and shorter than 2.25 cm, an electric field for separation is in a range of 350 to 550 V/cm,

(D) in the capillary channel for sample analysis, in a case where the cross-sectional shape is rectangular having a width and a depth of 60 $\mu$m each, when the distance from the electrophoresis starting point to the detecting point is 0.25 cm or longer and shorter than 0.75 cm, an electric field for separation is 150 V/cm or less,
0.75 cm or longer and shorter than 1.25 cm, an electric field for separation is in a range of 150 to 400 V/cm,
1.25 cm or longer and shorter than 1.75 cm, an electric field for separation is in a range of 250 to 450 V/cm,
1.75 cm or longer and shorter than 2.25 cm, an electric field for separation is in a range of 300 to 500 V/cm.

The protein in blood is considered as an analysis item, and the analysis item is hemoglobin.

The hemoglobin is at least one of hemoglobin A1c (HbA1c) and hemoglobin F (HbF).

Preferably, in the capillary electrophoresis analysis apparatus of the present invention, the hemoglobin A1e (HbA1c) is considered as an analysis item, and a hemoglobin A1c concentration (HbA1c concentration) is calculated as an analysis of the hemoglobin A1c (HbA1c).

Preferably, in the capillary electrophoresis analysis apparatus of the present invention, the sample is a sample prepared by subjecting the blood to a hemolysis treatment, and the hemoglobin in the sample that is prepared by subjecting the blood to the hemolysis treatment is analyzed.

In the analysis method of the present invention, an inner wall surface of the capillary channel for sample analysis may have an ionic functional group, pH of the electrophoresis running buffer may be in a range of 4.0 to 6.0, and an electroosmotic flow generated at the time of voltage application may be 3 cm/min or more.

In the analysis method of the present invention, the electrophoresis running buffer contains sulfated polysaccharide.

In the analysis method of the present invention, the sulfated polysaccharide is a chondroitin sulfate.

With respect to the analysis method of the present invention, any one of the following conditions (A) to (D) is satisfied:

(A) in the capillary channel for sample analysis, in a case where the cross-sectional shape is rectangular having a width and a depth of 30 μm each, when the distance from the electrophoresis starting point to the detecting point is 0.25 cm or longer and shorter than 0.75 cm, an electric field for separation is 300 V/cm or less,
0.75 cm or longer and shorter than 1.25 cm, an electric field for separation is in a range of 300 to 600 V/cm,
1.25 cm or longer and shorter than 1.75 cm, an electric field for separation is in a range of 400 to 650 V/cm,
1.75 cm or longer and shorter than 2.25 cm, an electric field for separation is in a range of 450 to 700 V/cm,

(B) in the capillary channel for sample analysis, in a case where the cross-sectional shape is rectangular having a width and a depth of 40 μm each, when the distance from the electrophoresis starting point to the detecting point is 0.25 cm or longer and shorter than 0.75 cm, an electric field for separation is 250 V/cm or less,
0.75 cm or longer and shorter than 1.25 cm, an electric field for separation is in a range of 250 to 500 V/cm,
1.25 cm or longer and shorter than 1.75 cm, an electric field for separation is in a range of 375 to 550 V/cm,
1.75 cm or longer and shorter than 2.25 cm, an electric field for separation is in a range of 400 to 600 V/cm,

(C) in the capillary channel for sample analysis, in a case where the cross-sectional shape is rectangular having a width and a depth of 50 μm each, when the distance from the electrophoresis starting point to the detecting point is 0.25 cm or longer and shorter than 0.75 cm, an electric field for separation is 200 V/cm or less,
0.75 cm or longer and shorter than 1.25 cm, an electric field for separation is in a range of 200 to 450 V/cm,
1.25 cm or longer and shorter than 1.75 cm, an electric field for separation is in a range of 300 to 500 V/cm,
1.75 cm or longer and shorter than 2.25 cm, an electric field for separation is in a range of 350 to 550 V/cm,

(D) in the capillary channel for sample analysis, in a case where the cross-sectional shape is rectangular having a width and a depth of 60 μm each, when the distance from the electrophoresis starting point to the detecting point is 0.25 cm or longer and shorter than 0.75 cm, an electric field for separation is 150 V/cm or less,
0.75 cm or longer and shorter than 1.25 cm, an electric field for separation is in a range of 150 to 400 V/cm,
1.25 cm or longer and shorter than 1.75 cm, an electric field for separation is in a range of 250 to 450 V/cm,
1.75 cm or longer and shorter than 2.25 cm, an electric field for separation is in a range of 300 to 500 V/cm.

In the analysis method of the present invention, the protein in blood is considered as an analysis item, and the analysis item is hemoglobin.

In the analysis method of the present invention, the hemoglobin is at least one of hemoglobin A1c (HbA1c) and hemoglobin F (HbF).

Preferably, in the analysis method of the present invention, the hemoglobin A1c (HbA1c) is considered as an analysis item, and a hemoglobin A1c concentration (HbA1c concentration) is calculated as an analysis of the hemoglobin A1c (HbA1c).

Preferably, in the analysis method of the present invention, the sample is a sample prepared by subjecting the blood to a hemolysis treatment, and the hemoglobin in the sample that is prepared by subjecting the blood to the hemolysis treatment is analyzed.

Hereinafter, the capillary electrophoresis analysis apparatus of the present invention is explained in details.

As described above, the capillary electrophoresis analysis apparatus of the present invention is applicable as long as it comprises an electrophoresis chip, a voltage application unit, a liquid sending unit, and an absorbance measurement unit according to claim 5. Alternatively, for example, the capillary electrophoresis analysis apparatus of the present invention may not include the liquid sending unit.

In the capillary electrophoresis analysis apparatus of the present invention, as described above, the electrophoresis chip comprises a substrate, a plurality of liquid reservoirs, and a capillary channel.

The size of the electrophoresis chip is not particularly limited. For example, the maximum length thereof is in the range of 10 mm to 100 mm, the maximum width thereof is in the range of 10 mm to 60 mm, and the maximum thickness thereof is in the range of 0.3 mm to 5 mm. Preferably, the maximum length thereof is in the range of 30 mm to 70 mm. The maximum length of the electrophoresis chip is the length of the portion that is longest in the longitudinal direction of the electrophoresis chip. The maximum width of the electrophoresis chip is the length of the portion that is longest in the short side direction of the electrophoresis chip. The maximum thickness of the electrophoresis chip is the length of the portion that is longest in the direction (thickness direction) perpendicular to both the longitudinal direction and the short side direction of the electrophoresis chip.

In the capillary electrophoresis analysis apparatus of the present invention, the electrophoresis chip is applicable as long as it comprises a substrate, a plurality of liquid reservoirs, and a capillary channel, and other configurations are not particularly limited.

The substrate is not particularly limited, and examples thereof include a case where the substrate is composed of one piece of substrate, a case where an upper substrate and a lower substrate are laminated together, and the like. The material of the substrate is not particularly limited, and examples thereof include a glass material, a polymeric material, etc. The glass material is not particularly limited, and examples thereof include synthetic silica glass, borosilicate glass, fused silica, etc. The polymeric material is not particularly limited, and examples thereof include polymethylmethacrylate (PMMA), cycloolefin polymer (COP), polycarbonate (PC), polydimethylsiloxane (PDMS), polystyrene (PS), polylactic acid (PLA), etc.

The liquid reservoir is not particularly limited and examples thereof include a concave portion formed by laminating a bottom part of a through hole formed in the upper substrate with the lower substrate, and the like. The form of the liquid reservoir is not particularly limited and examples thereof include a cylinder, a quadrangular prism, a quadrangular pyramid, a cone, and the like. Further, the volume of each liquid reservoir is not particularly limited and is, for example, in the range of 1 to 1000 mm$^3$, and preferably in the range of 10 to 100mm$^3$. The volume of each liquid reservoir may all be the same or may each be different, and it is not particularly limited.

The capillary channel may be formed in the substrate or may be a capillary tube embedded in the substrate.

In the capillary channel, the cross-sectional shape perpendicular to a channel direction is not particularly limited, and examples thereof include circular, rectangular, ellipsoidal, etc. In a case where the cross-sectional shape of the capillary channel is circular, the diameter thereof is, for example, in the range of 1 $\mu$m to 1000 $\mu$m, and preferably in the range of 10 $\mu$m to 200 $\mu$m, although it is not particularly limited. In a case where the cross-sectional shape of the capillary channel is rectangular, although the width and the depth thereof are not particularly limited, for example, the width thereof is in the range of 10 $\mu$m to 200 $\mu$m and the depth thereof is in the range of 10 $\mu$m to 200 $\mu$m. In the capillary channel, the length thereof is not particularly limited and is, for example, in the range of 0.5 cm to 15 cm, and preferably in the range of 1 cm to 5 cm.

As described above, the capillary channel includes the capillary channel for sample analysis. In the capillary channel for sample analysis, in a case where the cross-sectional shape perpendicular to a channel direction is circular, the diameter thereof is, for example, in the range of 10 $\mu$m to 200 $\mu$m, and preferably in the range of 25 $\mu$m to 100 $\mu$m, although it is not particularly limited. In the capillary channel for sample analysis, in a case where the cross-sectional shape is rectangular, for example, the width thereof is in the range of 25 $\mu$m to 100 $\mu$m and the depth thereof is in the range of 25 $\mu$m to 100 $\mu$m. Further, in the present invention, the length of the capillary channel for sample analysis is not particularly limited and is, for example, in the range of 0.5 cm to 15 cm, and preferably in the range of 1 cm to 5 cm.

As described above, the capillary channel may be formed by the substrate or may be formed in the substrate by embedding a capillary tube therein. In the former case, the material of the capillary channel is the material of the substrate, for example. In the latter case, the material of the capillary channel is the material of the capillary tube embedded, for example. The material of the capillary channel is not particularly limited, and examples thereof include, as in the above, a glass material such as synthetic silica glass, borosilicate glass, fused silica, etc.; a polymeric material such as polymethylmethacrylate (PMMA), cycloolefin polymer (COP), polycarbonate (PC), polydimethylsiloxane (PDMS), polystyrene (PS), polylactic acid (PLA), polyethylene (PE), polytetrafluoroethylene (PTFE), polyetheretherketone (PEEK), etc; and the like. A commercially available product may be used as the capillary tube, for example.

Normally, an inner wall of the glass capillary channel is negatively charged. For example, such glass capillary channel can be positively charged at the inner wall surface thereof by introducing a cationic group. Normally, according to the presence or absence or types of a polar group in polymer, an inner wall of the polymer capillary channel is positively or negatively charged or in a condition of charge-free (nonpolar). The charge-free polymer capillary channel can be charged at the inner wall surface thereof by introducing a polar group.

In the present invention, as described above, the inner wall of the capillary channel for sample analysis preferably has an ionic functional group at the surface thereof. Examples of the ionic functional group include a cationic group and an anionic group.

In the present invention, the capillary channel for sample analysis having the cationic group at the inner wall surface may be formed by coating the inner wall of the capillary channel for sample analysis with a cationic group-containing compound. Hereinafter, coating of the cationic group-containing compound may be referred to as a cationic layer. By means of the coating of the cationic group-containing compound (the cationic layer), the inner wall of the capillary channel for sample analysis can be prevented from being adsorbed with positively-charged protein or the like in a sample, for example. Further, due to the coating of the cationic group-containing compound, an electroosmotic flow tends to be faster than the case of untreated. The cationic group-containing compound is not particularly limited and examples thereof include a compound containing the cationic group and a reactive group, and the like. For example, in a case where the capillary channel for sample analysis is made of glass or fused silica, a compound (silylation agent) containing a cationic group and silicon; or the like can be used as the compound containing the cationic group and the reactive group. Preferable examples of the cationic group include an amino group and an ammonium group, although it is not particularly limited. A preferable example of the cationic group-containing compound includes a silylation agent having at least one cationic group of an amino group and an ammonium group, although it is not particularly limited. The amino group may be a primary amino group, a secondary amino group, or a tertiary amino group.

Examples of the silylation agent include N-(2-diaminoethyl)-3-propyltrimethoxysilane, aminophenoxydimethylvinylsilane, 3-aminopropyldiisopropylethoxysilane, 3-aminopropylmethylbis(trimethylsiloxy)silane, 3-aminopropylpentamethyldisiloxane, 3-aminopropylsilanetriol, bis(p-aminophenoxy)dimethylsilane, 1,3-bis(3-aminopropyl)tetramethyldisiloxane, bis(dimethylamino)dimethylsilane, bis(dimethylamino)vinylmethylsilane, bis(2-hydroxyethyl)-3-aminopropyltriethoxysilane, 3-cyanopropyl(diisopropyl)dimethylaminosilane, (aminoethylaminomethyl)phenethyltrimethoxysilane, N-methylaminopropyltriethoxysilane, tetrakis(diethylamino)silane tris(dimethylamino)chlorosilane, tris(dimethylamino)silane, and the like.

With respect to the cationic group-containing compound, besides them, a compound in which a silicon atom in the silylation agent is substituted by titanium or zirconium may be used. One of the cationic group-containing compounds may be used alone or two or more of them may be used in combination.

For example, coating of the inner wall of the capillary channel for sample analysis using the silylation agent is carried out as follows. First, a treatment solution is prepared by dissolving or dispersing the silylation agent to an organic solvent. As the organic solvent used for preparing the treatment solution, dichloromethane, toluene, methanol, acetone, and the like can be used. A concentration of the silylation agent in the treatment solution is not particularly limited. This treatment solution is passed through a capillary channel made of glass or fused silica, and heated. Due to this heating, the silylation agent is covalently-bonded to the inner wall of the capillary channel. As a result, a cationic group is arranged on the inner wall of the capillary channel. Thereafter, as an aftertreatment, the organic solvent is washed away with at least one of an acid solution such as phosphoric acid, and the like; an alkaline solution; and a surfactant solution, and the inside of the capillary channel is thereby washed. This washing is preferably performed, although it is optional. A commercially-available product can be used as the capillary channel, the inner wall of which is coated with the silylation agent.

The capillary channel for sample analysis having the anionic group at the inner wall surface thereof may be formed by coating the inner wall of the capillary channel for sample analysis with an anionic group-containing compound, for example. Hereinafter, coating of the anionic group-containing compound may be referred to as an anionic layer. By means of the coating of the anionic group-containing compound (the anionic layer), the inner wall of the capillary channel for sample analysis can be prevented from being adsorbed with negatively-charged protein or the like in a sample, for example. Further, due to the coating of the anionic group, an electroosmotic flow tends to be faster than the case of untreated. In a case of the coating of the anionic group-containing compound, a direction of the electroosmotic flow is the opposite direction from that in a case of the coating of the cationic group-containing compound. Further, since the sample and the anionic group-containing compound present in an electrophoresis running buffer form a complex and the complex is subjected to an electrophoresis, separation efficiency is increased relative to a case where the sample is independently subjected to an electrophoresis. As a result, more brief and more accurate analysis can be performed. For example, as the anionic group-containing compound which forms a complex with the sample, an anionic group-containing polysaccharide is preferable.

Examples of the anionic group-containing polysaccharide include sulfated polysaccharide, carboxylated polysaccharide, sulfated polysaccharide, phosphorylated polysaccharide, and the like. Among them, sulfated polysaccharide and carboxylated polysaccharide are preferable. As the sulfated polysaccharide, chondroitin sulfate, heparin, and the like are preferable, and chondroitin sulfate is more preferable. As the carboxylated polysaccharide, algin acid or its salt (for example, alginate sodium) is preferable. There are seven types of chondroitin sulfate such as chondroitin sulfate A, chondroitin sulfate B, chondroitin sulfate C, chondroitin sulfate D, chondroitin sulfate E, chondroitin sulfate H, and chondroitin sulfate K. Although any one of them may be used, chondroitin sulfate C is preferable.

The anionic layer may be laminated onto the inner wall of the capillary channel for sample analysis via an intercalated layer, for example. The intercalated layer is not particularly limited, and can be formed using the cationic group-containing compound, for example. The anionic layer laminated via the intercalated layer may be formed by making the inner wall of the capillary channel for sample analysis coated with the cationic group-containing compound in contact with liquid containing the anionic group-containing compound. In this case, liquid for forming the anionic group-containing compound may separately be prepared. However, from the viewpoint of operation efficiency, it is preferable that an electrophoresis running buffer prepared by adding the anionic group-containing compound is passed through the capillary channel, the intercalated layer is formed on the inner wall thereof.

In the present invention, it is not limited to the capillary channel for sample analysis as described above, inner walls of other capillary channels formed on the substrate may have the ionic functional group, or inner walls of all capillary channels may have the ionic functional group, for example. The other capillary channels having the ionic functional group at the inner walls thereof can be formed in the same manner as the capillary channel for sample analysis described above.

In the present invention, a sample containing protein in blood to be analyzed and an electrophoresis running buffer are introduced into the capillary channel for sample analysis.

In the present invention, although it is not particularly limited, an electrophoresis running buffer containing organic acid is preferable as the electrophoresis running buffer. The organic acid is not particularly limited, and examples thereof include maleic acid, tartaric acid, succinic acid, fumaric acid, phthalic acid, malonic acid, malic acid, etc. Further, although it is not particularly limited, the electrophoresis running buffer preferably contains weak base. The weak base is not particularly limited, and examples thereof include arginine, lysine, histidine, tris, etc. The electrophoresis running buffer is not particularly limited, and examples thereof include, N-(2-acetamido)iminodiacetic acid (ADA) buffer solution, morpholinoethanesulfonic acid (MES) buffer solution, bis(2-hydroxyethyl)iminotris(hydroxymethyl)methane (Bis-Tris) buffer solution, piperazine-1,4-bis(2-ethanesulfonic acid)(PIPES) buffer solution, N-(2-acetamido)-2-aminoethanesulfonic acid (ACES) buffer solution, 2-Hydroxy-3-morpholinopropanesulfonic acid (MOPSO) buffer solution, N,N-bis(2-hydroxyethyl)-2-aminoethanesulfonic acid (BES) buffer solution, 2-[4-(2-hydroxyethyl)-1-piperazinyl]ethanesulfonic acid (HEPES) buffer solution, N-tris(hydroxymethyl)methyl-2-aminoethanesulfonic acid (TES) buffer solution, phosphoric acid buffer solution, etc. The pH of the electrophoresis running buffer is not particularly limited and is for example in the aforementioned range, and preferably in the range of 4.5 to 6.0.

In the present invention, as described above, the anionic group-containing compound is preferably added to the electrophoresis running buffer. The anionic group-containing compound is not particularly limited, and examples thereof include the aforementioned anionic group-containing compounds, and the like. The concentration of the anionic group-containing compound contained in the electrophoresis running buffer is not particularly limited, and is, for example, in the range of 0.001 to 10 wt%, and preferably in the range of 0.1 to 5 wt%.

In the present invention, for example, a surfactant may be added to the electrophoresis running buffer. The surfactant is not particularly limited and examples thereof include a betaine type ampholytic surfactant, a nonionic surfactant, and the like. The betaine type ampholytic surfactant is not particularly limited and examples thereof include a carboxybetaine type surfactant, a sulfobetaine type surfactant, and the like. Examples of the carboxybetaine type surfactant include N,N-dimethyl-N-alkyl-N-carboxy alkylene ammonium betaine, and the like. Examples of the sulfobetaine type surfactant include N,N,N-trialkyl-N-sulfoalkyleneammonium betaine, and the like. Specific examples of the sulfobetaine type surfactant include palmityl sulfobetaine, and the like.

In the present invention, an anionic chaotropic ion may be added to the electrophoresis running buffer, for example: In the present invention, the anionic chaotropic ion includes a salt containing an anionic chaotropic ion, a substance generating an anionic chaotropic ion by ionization, and the like. Examples of the salt include acid salt, neutral salt, and basic salt. In the present invention, the anionic chaotropic ion is not particularly limited, and examples thereof include perchloric acid, thiocyanic acid, potassium iodide, potassium bromide, trichloroacetic acid, trifluoroacetic acid, and the like. The concentration of the anionic chaotropic ion is not particularly limited and is, for example, in the range of 1 to 3000 mmol/L, preferably in the range of 5 to 100 mmol/L, and more preferably in the range of 10 to 50 mmol/L.

In the present invention, examples of a sample that contains the protein in blood to be analyzed include whole blood, a sample prepared by subjecting blood to a hemolysis treatment, and the like. Among them, in the present invention, it is preferable that protein in the sample that is prepared by subjecting blood to a hemolysis treatment is analyzed. Examples of the hemolysis treatment include a surfactant treatment, an osmotic pressure treatment, a sonication treatment, a freeze/thaw treatment, a pressure treatment, etc., and the surfactant treatment, the osmotic pressure treatment, and the sonication treatment are preferable.

The surfactant treatment is not particularly limited and, for example, it may be a treatment in which whole blood is hemolyzed with a diluent to which a surfactant is added. Examples of the surfactant include the aforementioned surfactants, saponin, etc. The osmotic pressure treatment is not particularly limited, it may be a treatment in which whole blood is hemolyzed with a solution that is adjusted to have low osmotic pressure. The solution is not particularly limited, and examples thereof include distilled water, a diluent that is adjusted to have low osmotic pressure, and the like, and the distilled water is preferable. The sonication treatment is not particularly limited, and an ultrasonic processor can be used, for example. In the present invention, the diluent is not particularly limited, and examples thereof include distilled water, the aforementioned electrophoresis running buffer, and the like.

In the present invention, the protein in blood is protein contained in blood. The protein in blood is not particularly limited, and examples thereof include hemoglobin (Hb), albumin (Alb), globulin ($\alpha1$, $\alpha2$, $\beta$, $\Gamma$ globulin), fibrinogen, etc.

The hemoglobin is not particularly limited, and examples thereof include normal hemoglobin (HbA0), glycated hemoglobin, modified hemoglobin, fetal hemoglobin (HbF), etc. Examples of the glycated hemoglobin include hemoglobin A1a (HbA1a), hemoglobin A1b (HbA1b), hemoglobin A1c (HbA1c), GHbLys, etc. Examples of the hemoglobin A1c include stable HbA1c, unstable HbA1c, etc. Examples of the modified hemoglobin include carbamoylated Hb, acetylated Hb, etc.

In the capillary electrophoresis analysis apparatus of the present invention, the protein in blood serves as an analysis item. The analysis item is not particularly limited, and hemoglobin is preferable among the aforementioned protein in blood, for example.

In the present invention, the analysis item is not particularly limited, and examples thereof include a ratio of various hemoglobin, a hemoglobin A1c concentration, an albumin concentration, a globulin concentration, an albumin/globulin ratio, etc.

In the present invention, an electrophoresis of the sample is performed from an electrophoresis starting point toward a detecting point by an electroosmotic flow generated due to voltage application to the electrode. Then, at the detecting point on the capillary channel for sample analysis, an absorbance of protein in blood in the sample that is subjected to the electrophoresis is measured.

In the present invention, the electrophoresis starting point is a point, which is placed on the capillary channel for sample analysis, where an electrophoresis of the sample that is introduced into the capillary channel for sample analysis is started upon voltage application. The electrophoresis starting point is not particularly limited, and examples thereof include a boundary between a liquid reservoir, into which the sample is introduced, and the capillary channel for sample analysis, and the like. Further, in the present invention, as described above, the detecting point is a point where an absorbance of protein in blood in the sample that is subjected to the electrophoresis in the capillary channel for sample analysis is measured. The detecting point is placed on the capillary channel for sample analysis and the distance from the electrophoresis starting point is preferably in the range that will be described below, although it is not particularly limited. In the present invention, the distance from the electrophoresis starting point to the detecting point (separation length) is, for example, in the range of 0.5 cm to 15 cm, and preferably in the range of 1 cm to 5 cm, although it is not particularly limited.

In the present invention, the voltage is, for example, in the range of 0.075 kV to 20 kV, and preferably in the range of 0.3 kV to 5 kV, although it is not particularly limited. In the present invention, the electroosmotic flow is, for example, in the range of 3cm/min to 15 cm/min, and preferably in the range of 8 cm/min to 12 cm/min, although it is not particularly limited.

In the present invention, a measurement wave length of the absorbance is, for example, in the range of 260 nm to 300 nm or 380 nm to 450 nm, and preferably in the range of 400 nm to 430 nm.

In the present invention, an analysis time of the protein in blood is a time required for separating the protein in blood that is contained in the sample to be analyzed and completing the detection thereof, for example. More specifically, the analysis time of the protein in blood is a time from the start of the voltage application to the electrode to the completion of detection of all proteins in blood to be analyzed, for example.

In the present invention, the analysis time of the protein in blood exceeds 0 second and is 35 seconds or shorter, preferably exceeds 0 second and is 30 seconds or shorter, more preferably exceeds 0 second and is 25 seconds or shorter, and yet more preferably exceeds 0 second and is 20 seconds or shorter.

Preferably, in the present invention, the distance from the electrophoresis starting point to the detecting point and the electric field for separation are set in the aforementioned predetermined range in accordance with the width and the depth of the capillary channel. In the present invention, the electric field for separation is a voltage to be applied to an interelectrode distance per cm. In the present invention, by setting the distance from the electrophoresis starting point to the detecting point and the electric field for separation in the aforementioned predetermined range, the protein in blood can be analyzed in a short time such as 35 seconds or shorter. In the present invention, the predetermined range is not limited to the aforementioned range, and can be adjusted suitably according to types of electrophoresis running buffer, the coating methods of the ionic functional group to the capillary channel, and the like.

Although it is not particularly limited, the capillary electrophoresis analysis apparatus may further comprise a quantitative dispensing unit, a stirring unit, a stray light removing unit, a position adjustment unit, and the like.

Next, the capillary electrophoresis analysis apparatus of the present invention and the analysis method of the present invention using the same are explained with Examples. However, the capillary electrophoresis analysis apparatus of the present invention and the analysis method of the present invention using the same are not limited to the following Examples.

Brief Description of Drawings

FIG. 1A is a plan view showing an example of an electrophoresis chip in the capillary electrophoresis analysis apparatus of the present invention. FIG. 1B is a cross-sectional view of the electrophoresis chip shown in FIG. 1A viewed in the direction of line I-I.

FIG. 2 is a schematic view showing an example of the capillary electrophoresis analysis apparatus of the present invention.

FIG. 3A is a plan view showing another example of an electrophoresis chip in the capillary electrophoresis analysis apparatus of the present invention. FIG. 3B is a cross-sectional view of the electrophoresis chip shown in FIG. 3A viewed in the direction of line I-I. FIG. 3C is a cross-sectional view of the electrophoresis chip shown in FIG. 3A viewed in the direction of line II-II.

FIG. 4A is a plan view showing yet another example of an electrophoresis chip in the capillary electrophoresis analysis apparatus of the present invention. FIG. 4B is a perspective view of the electrophoresis chip shown in FIG. 4A.

FIG. 5 is a schematic view showing another example of the capillary electrophoresis analysis apparatus of the present invention.

FIG. 6 is a graph showing an analysis result of hemoglobin in an example of the analysis method of the present invention.

FIG. 7 is a graph showing an analysis result of hemoglobin in another example of the analysis method of the present invention.

FIG. 8 is a graph showing an analysis result of hemoglobin in yet another example of the analysis method of the present invention.

FIG. 9 is a graph showing an analysis result of hemoglobin in yet another example of the analysis method of the present invention.

FIG. 10 is a graph showing an analysis result of hemoglobin in yet another example of the analysis method of the present invention.

FIG. 11 is a graph showing an analysis result of hemoglobin in analysis method of a comparative example.

FIG. 12 is a graph showing an analysis result of hemoglobin in analysis method of another comparative example.

[Embodiment 1]

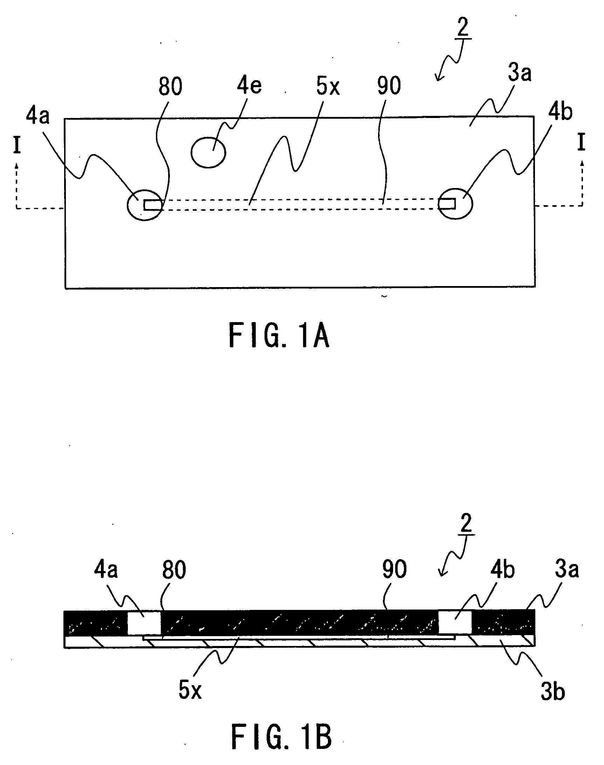

An electrophoresis chip used for the capillary electrophoresis analysis apparatus of this embodiment is shown in FIG. 1. FIG. 1A is a plan view of the electrophoresis chip of this embodiment. FIG. 1B is a cross-sectional view of the electrophoresis chip shown in FIG. 1A viewed in the direction of line I-I. For easier understanding, the size, proportions and like features of each component in the illustrations are different from the actual features of each component. As shown in FIG. 1, the electrophoresis chip 2 of this embodiment is composed of a lower substrate 3b and an upper substrate 3a, the upper substrate 3a being laminated onto the lower substrate 3b. Three through-holes are formed in the upper substrate 3a. The bottom parts of the three through-holes formed in the upper substrate 3a are sealed with the lower substrate 3b and, thus three fluid reservoirs 4a, 4b, and 4e are formed. A groove having a shape of "I" is formed on the lower substrate 3b. The upper part of the groove having a shape of "I" formed on the lower substrate 3b is sealed with the upper substrate 3a and, thus a capillary channel for sample analysis 5x is formed. The liquid reservoir 4a and the liquid reservoir 4b are in communication with each other via the capillary channel for sample analysis 5x. In contrast, the liquid reservoir 4e is not in communication with the capillary channel for sample analysis 5x and is provided as an independent liquid reservoir. An end of the capillary channel for sample analysis 5x at the liquid reservoir 4a side serves as an electrophoresis starting point 80. Further, a point on the capillary channel for sample analysis 5x between the liquid reservoir 4a and the liquid reservoir 4b serves as a detecting point 90. The electrophoresis chip 2 of this embodiment is rectangular parallelepiped. However, the present invention is not limited thereto. In the present invention, the electrophoresis chip may be in any form as long as it does not adversely affect the analysis of the sample which will be described below. Furthermore, the electrophoresis chip 2 of this embodiment includes two substrate pieces (an upper substrate 3a and a lower substrate 3b). However, the present invention is not limited thereto. In the present invention, the electrophoresis chip may be composed of a single-piece substrate.

In the electrophoresis chip 2 of this embodiment, the length and the width of the upper substrate 3a correspond to the maximum length and the maximum width of the whole electrophoresis chip described above. Therefore, the length and the width of the upper substrate 3a are arranged to be identical to the maximum length and the maximum width of the whole electrophoresis chip described above, respectively. The thickness of the upper substrate 3a in the electrophoresis chip 2 of this embodiment can be set suitably according to the volume of the plural liquid reservoirs 4a, 4b, and 4e, however is, for example in the range of 0.1 mm to 3 mm, and preferably in the range of 1 mm to 2mm.

In the electrophoresis chip 2 of this embodiment, the length and the width of the lower substrate 3b are the same as the length and the width of the upper substrate 3a, respectively. The thickness of the lower substrate 3b is not particularly limited, however is, for example, in the range of 0.1 mm to 3 mm, and preferably in the range of 0.1 mm to 1 mm.

The material of the upper substrate 3a and the lower substrate 3b is not particularly limited as long as it does not adversely affect the measurement of the absorbance. As the material of the upper substrate 3a and the lower substrate 3b, the aforementioned materials and the like can be used.

With respect to the width and the depth of the capillary channel for sample analysis 5x, for example, the width thereof is in the range of 25 $\mu$m to 100 $\mu$m and the depth thereof is in the range of 25 $\mu$m to 100 $\mu$m. Further, the distance from the electrophoresis starting point 80 to the detecting point 90 is, for example, in the range of 0.5 cm to 15 cm, and preferably in the range of 1 cm to 5 cm.

The volume of the liquid reservoirs 4a, 4b, and 4e are as described above. In FIG. 1, liquid reservoirs 4a, 4b, and 4e are cylinder. However, the present invention is not limited thereto. In the present invention, as described above, the liquid reservoirs 4a, 4b, and 4e may be in any form.

In the electrophoresis chip 2 of this embodiment, the maximum thickness of the chip is the sum of the thickness of the upper substrate 3a and the lower substrate 3b. The thickness of the whole chip is as described above.

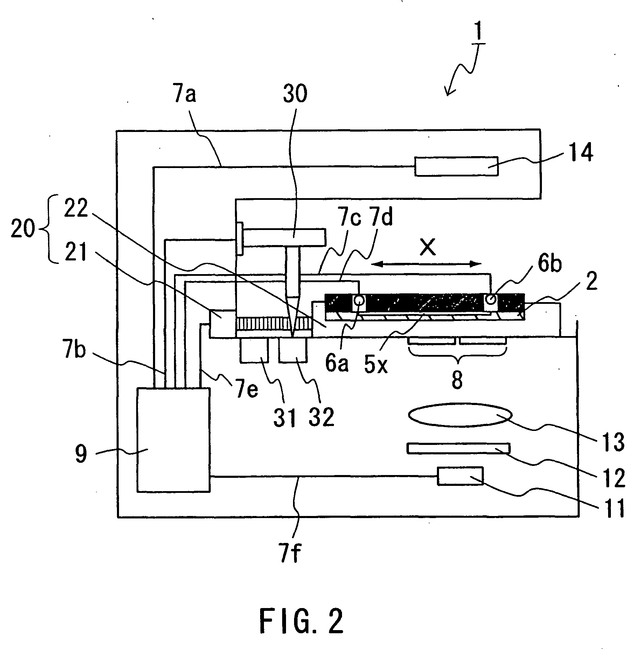

A capillary electrophoresis analysis apparatus of this embodiment is shown in FIG. 2. As shown in FIG. 2, this capillary electrophoresis analysis apparatus 1 includes the aforementioned electrophoresis chip 2, electrodes 6a and 6b, electric wires 7a to 7f, a slit 8, a control unit 9, a light source 11, an optical filter 12, a collecting lens 13, a detection unit 14, an electrophoresis chip transfer unit 20, a quantitative dispensing unit 30, a diluent 31, and an electrophoresis running buffer 32 as main components. The electrophoresis chip transfer unit 20 contains a drive unit 21 and a stage 22. The electrophoresis chip 2 is arranged on the stage 22. The electrodes 6a and 6b are arranged in the liquid reservoirs 4a and 4b of the electrophoresis chip 2, respectively. The detection unit 14, the quantitative dispensing unit 30, the electrodes 6a and 6b, the electrophoresis chip transfer unit 20, and the light source 11 are connected to the control unit 9 via the electric wires 7a to 7f, respectively. The control unit 9 controls power supply or the like to the aforementioned components which are connected thereto via the electric wires 7a to 7f.

In the capillary electrophoresis analysis apparatus 1 of this embodiment, the stage 22 is movable in a horizontal biaxial direction (an X-Y direction) by the drive unit 21 that is connected to an end thereof. The X direction and the Y direction vertically intersect on the horizontal surface. Thereby, the position of the electrophoresis chip 2 can be adjusted. Due to adjustment of the position of the electrophoresis chip 2, the detecting point 90 can accurately be irradiated with the light flux of specific wavelength. Further, the quantitative dispensing unit 30 can perform a quantitative analysis of the diluent 31 and the electrophoresis running buffer 32, respectively, and can dispense them to the liquid reservoir 4a or the liquid reservoir 4e of the electrophoresis chip 2. By applying the voltage between the electrodes 6a and 6b, an electrophoresis of a sample that is introduced into the capillary channel for sample analysis 5x can be performed. Then, the light emitted from the light source 11 is dispersed into specific wavelength by the optical filter 12 and converged by the collecting lens 13, as well as the amount of light is increased and the stray light is removed by the slit 8, and then the sample at the detecting point 90 on the capillary channel for sample analysis 5x of the electrophoresis chip 2 is irradiated. The transmitted light of the light irradiated on the detecting point 90 is detected by the detection unit 14 and an absorbance is measured. Thereby, protein in blood to be analyzed contained in the sample can be analyzed.

The method of producing the electrophoresis chip 2 of the capillary electrophoresis analysis apparatus 1 of this embodiment is not particularly limited and conventionally known methods can suitably be applied.

Next, the method of analyzing the protein in blood using the capillary electrophoresis analysis apparatus 1 of this embodiment is explained.

First, the electrophoresis running buffer 32 is prepared. The electrophoresis running buffer 32 may be the aforementioned electrophoresis running buffer and it is not particularly limited. However, for example, a solution prepared by adding chondroitin sulfate C in a proportion of 0.8 wt% to a solution of 100 mmol/L containing fumaric acid and arginine acid, the solution being adjusted to pH 4.8, can be used. Next, the electrophoresis chip 2 is attached to the stage 22 and disposed in the capillary electrophoresis analysis apparatus 1. Then, the electrophoresis running buffer 32 is injected into the liquid reservoir 4a using the quantitative dispensing unit 30. Further, the pressure in the capillary channel for sample analysis 5x is reduced by a pressure reduction pump (not shown) that is connected to the liquid reservoir 4b and the capillary channel for sample analysis 5x is filled with the electrophoresis running buffer 32.

Next, the diluent 31 is injected into the liquid reservoir 4e using the quantitative dispensing unit 30. Further, a human whole blood is added to the reservoir 4e as a sample and is stirred by pipetting, and thus a mixture of the sample and the diluent 31 is prepared. As the diluent 31, distilled water or the like can be used. Subsequently, the mixture is injected into the liquid reservoir 4a. Then, the voltage is applied to the electrodes 6a and 6b, which are respectively arranged in the liquid reservoirs 4a and 4b, thereby creating a potential difference between both ends of the capillary channel for sample analysis 5x. The sample is thereby moved from the electrophoresis starting point 80 to the liquid reservoir 4b side. The voltage is not particularly limited, however is, for example, in the range of 0.5 to 20kV. As described above, the electric field for separation of the capillary channel for sample analysis 5x due to the voltage application can be set suitably according to the distance from the electrophoresis starting point to the detecting point and the width and the depth of the capillary channel. The electric field for separation of the capillary channel for sample analysis 5x is, for example, in the range of 150 V/cm to 700 V/cm, and preferably in the range of 300 V/cm to 600 V/cm.

Next, in the same manner as described above, the light is dispersed and collected, and then the detecting point 90 is irradiated with the light (wave length of 415 nm), from which a stray light is further removed. Then, the transmitted light at the detecting point 90 is detected by the detection unit 14 and the absorbance of the protein in blood in the sample is measured. Electropherogram is made that indicates relationship between degree of the obtained absorbance and analysis time (from start of electrophoresis to detection).

[Embodiment 2]

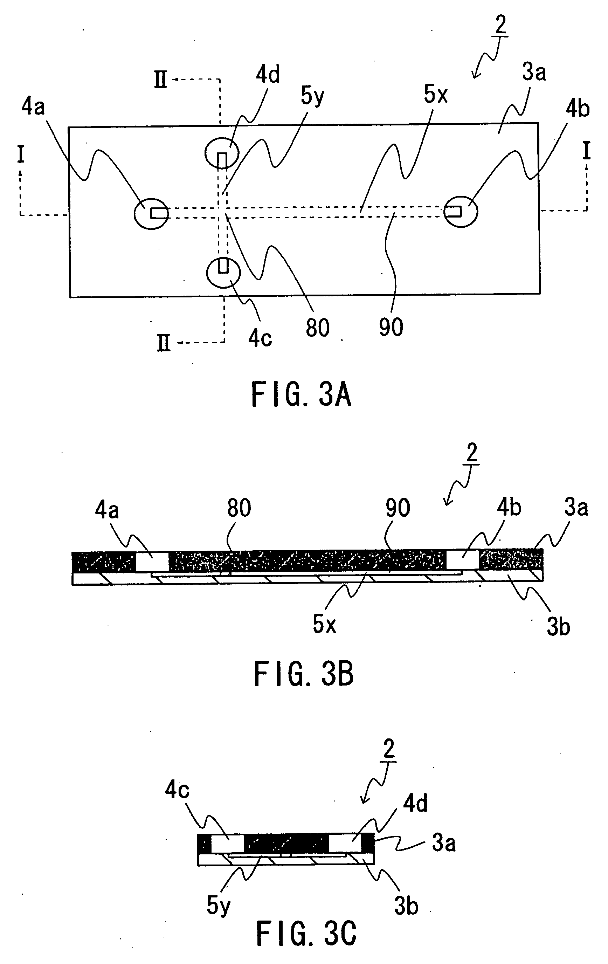

An electrophoresis chip used for the capillary electrophoresis analysis apparatus of this embodiment is shown in FIG. 3. In FIG. 3, the portions that are identical to those in FIG. 1 are given the same numbers and symbols. FIG. 3A is a plan view of the electrophoresis chip of this embodiment, FIG. 3B is a cross-sectional of the electrophoresis chip shown in FIG. 3A viewed in the direction of line I-I, and FIG. 3C is a cross-sectional view of the electrophoresis chip shown in FIG. 3A viewed in the direction of line II-II.

As shown in FIG. 3, the electrophoresis chip 2 of this embodiment is composed of a lower substrate 3b and an upper substrate 3a, the upper substrate 3a being laminated onto the lower substrate 3b. Plural through-holes (four in this embodiment) are formed in the upper substrate 3a. The bottom parts of the four through-holes formed in the upper substrate 3a are sealed with the lower substrate 3b and, thereby four liquid reservoirs 4a to 4d are formed. A cross-shaped groove is formed on the lower substrate 3b. By sealing the upper part of the cross-shaped groove formed on the lower substrate 3b with the upper substrate 3a, a capillary channel for sample analysis 5x and a capillary channel for sample introduction 5y are formed. The liquid reservoir 4a and the liquid reservoir 4b are in communication with each other via the capillary channel for sample analysis 5x. The liquid reservoir 4c and the liquid reservoir 4d are in communication with each other via the capillary channel for sample introduction 5y. The capillary channel for sample analysis 5x and the capillary channel for sample introduction 5y intersect. The capillary channel for sample analysis 5x and the capillary channel for sample introduction 5y are in communication with each other at the intersection. The intersection serves as an electrophoresis starting point 80. Further, a point on the capillary channel for sample analysis 5x between the liquid reservoir 4a and the liquid reservoir 4b serves as a detecting point 90. In the electrophoresis chip 2 of this embodiment, the maximum length of the capillary channel for sample analysis 5x is different from that of the capillary channel for sample introduction 5y. However, the present invention is not limited thereto. In the present invention, the maximum length of the capillary channel for sample analysis 5x of the electrophoresis chip may be the same as the maximum length of the capillary channel for sample introduction 5y of the electrophoresis chip.

The electrophoresis chip 2 of this embodiment has the same configuration as the electrophoresis chip shown in FIG. 1 except that the liquid reservoirs 4c and 4d and the capillary channel for sample introduction 5y are formed as well as the liquid reservoir 4e is not formed. The width and the depth of the capillary channel for sample introduction 5y are the same as the width and the depth of the capillary channel for sample analysis 5x. The distance from the electrophoresis starting point 80 to the detecting point 90 is the same as that of the electrophoresis chip shown in FIG. 1. The volume and the form of the liquid reservoirs 4c and 4d are the same as that of the electrophoresis chip shown in FIG. 1.

A capillary electrophoresis analysis apparatus of this embodiment has the same configuration as the capillary electrophoresis analysis apparatus shown in FIG. 2 except that the electrophoresis chip 2 is the electrophoresis chip shown in FIG. 3 instead of the electrophoresis chip shown in FIG. 1 as well as electrodes 6c and 6d (not shown) are arranged in the liquid reservoirs 4c and 4d of the electrophoresis chip 2.

Next, the method of analyzing the protein in blood using the capillary electrophoresis analysis apparatus 1 of this embodiment is explained.

First, the electrophoresis chip 2 is attached to a stage 22 and disposed in the capillary electrophoresis analysis apparatus 1 of this embodiment. Subsequently, in the same manner as in Embodiment 1, the electrophoresis running buffer 32 is injected into the liquid reservoir 4a using the quantitative dispensing unit 30. Next, in the same manner as in Embodiment 1, the pressure in the capillary channel for sample analysis 5x is reduced by a pressure reduction pump (not shown) that is connected to the liquid reservoir 4b, and the capillary channel for sample analysis 5x is filled with the electrophoresis running buffer 32. Then, the electrophoresis running buffer 32 is injected into the liquid reservoir 4c using the quantitative dispensing unit 30. Further, the pressure in the capillary channel for sample introduction 5y is reduced by a pressure reduction pump (not shown) that is connected to the liquid reservoir 4d, and the capillary channel for sample introduction 5y is filled with the electrophoresis running buffer 32.

Next, the diluent 31 is injected into the liquid reservoir 4c using the quantitative dispensing unit 30. Further, a human whole blood is added thereto as a sample and is stirred by pipetting. Then, the voltage is applied to the electrodes 6c and 6d, thereby creating a potential difference between both ends of the capillary channel for sample introduction 5y. The sample is thereby moved to the intersection of the capillary channel for sample analysis 5x and the capillary channel for sample introduction 5y. The voltage applied between the electrodes 6c and 6d is not particularly limited, however is, for example, in the range of 0.5 to 20 kV.

Next, the voltage is applied to the electrodes 6a and 6b, thereby creating a potential difference between both ends of the capillary channel for sample analysis 5x. The sample is thereby moved from the electrophoresis starting point 80 to the liquid reservoir 4b side. The voltage is not particularly limited, however is, for example, in the range of 0.5 to 20 kV. As described above, the electric field for separation of the capillary channel for sample analysis 5x due to the voltage application can be set suitably according to the distance from the electrophoresis starting point to the detecting point and the width and the depth of the capillary channel. However, the electric field for separation of the capillary channel for sample analysis 5x is, for example, in the same range as Embodiment 1.

Next, in the same manner as in Embodiment 1, the light is dispersed and collected, and then the detecting point 90 is irradiated with the light (wave length of 415 nm), from which a stray light is further removed. Then, the transmitted light at the detecting point 90 is detected using the detection unit 14 and the absorbance of the protein in the sample is measured. Electropherogram is made that indicates relationship between degree of the obtained absorbance and analysis time (electrophoresis time).

[Embodiment 3]

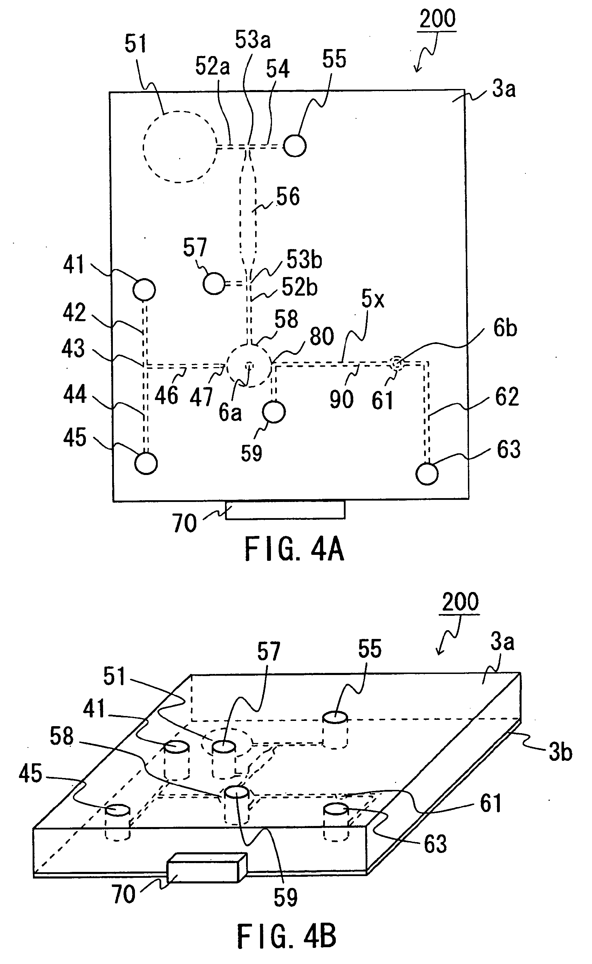

An electrophoresis chip used for the capillary electrophoresis analysis apparatus of this embodiment is shown in FIG. 4. In FIG. 4, the portions that are identical to those in FIG. 1 and FIG. 3 are given the same numbers and symbols. FIG.4Ais a plan view of the electrophoresis chip of this embodiment, and FIG. 4B is a perspective view of the electrophoresis chip of this embodiment. As shown in FIG. 4, the electrophoresis chip 200 of this embodiment includes a laminated body, in which an upper substrate 3a is laminated onto a lower substrate 3b, and a connector 70. The connector 70 is arranged on a side surface of the laminated body. A wiring pattern (not shown) is formed on the lower substrate 3b. Six through-holes are formed in the upper substrate 3a. The bottom parts of the six through-holes are sealed with the lower substrate 3b, and thereby six liquid reservoirs are formed. The six liquid reservoirs serve as a sample introduction portion 41, a drain 45, a drain 55, a drain 57, a drain 59, and a drain 63, respectively. Further, three concave portions of various sizes are formed at the bottom surface of the upper substrate 3a. Openings of two concave portions out of the three concave portions are sealed with the lower substrate 3b, and thereby two liquid reservoirs are formed. The two liquid reservoirs serve as a reagent reservoir 51 and a diluent reservoir 58, respectively. An electrophoresis running buffer is sealed in the reagent reservoir 51. An electrode 6a connected to a wiring of the wiring pattern is arranged in the diluent reservoir 58, and a stirring bar (not shown) is sealed in the diluent reservoir 58. An opening of the other one of the concave portion out of the three concave portions is sealed with the lower substrate 3b, and thereby an electrode arrangement portion 61 is formed. An electrode 6b connected to a wiring of the wiring pattern is arranged in the electrode arrangement portion 61. Further, plural grooves are formed on the bottom surface of the upper substrate 3a. Openings of the plural grooves are sealed with the lower substrate 3b, and thereby channels are formed, through which the six reservoirs and the three concave portions are in communication with one another. A capillary channel, through which the diluent reservoir 58 and the electrode arrangement portion 61 are in communication with each other, serves as the capillary channel for sample analysis 5x. An end portion of the capillary channel for sample analysis 5x at the diluent reservoir 58 side serves as an electrophoresis starting point 80. Further, a point on the capillary channel for sample analysis 5x serves as a detecting point 90. Details of channels other than the capillary channel for sample analysis 5x will be described later.

The sample introduction portion 41 is in communication with the drain 45 via a sample introduction channel 42, a branching portion 43, and an overflow channel 44 in order. Further, the sample introduction portion 41 is also in communication with the diluent reservoir 58 from the branching portion 43 via a sample measurement channel 46. The sample introduction portion 41 is an introduction opening for introducing a sample, which contains protein in blood to be analyzed, into an electrophoresis chip. At an end portion of the sample measurement channel 46 at the diluent reservoir 58 side, an orifice 47 having a narrow channel cross-sectional area is formed.

In the electrophoresis chip 200 of this embodiment, the sample is measured and introduced into the electrophoresis chip in the following manner. First, after introducing a sample into the sample introduction portion 41, air is sucked from the drain 45 with a pressure reduction pump (not shown) or the like. Due to the suction, a sample that exceeds the volume of the sample measurement channel 46 between the branching portion 43 and the orifice 47 flows into the overflow channel 44. Subsequently, the drain 45 is closed and an air is discharged from the sample introduction portion 41 with a pressure pump (not shown) or the like. Thereby, a sample corresponding to the volume of the sample measurement channel 46 stored therein is measured and introduced into the diluent reservoir 58.

The reagent reservoir 51 is in communication with the drain 55 via a reagent introduction channel 52a, a branching portion 53a, and an overflow channel 54 in order. Further, the reagent reservoir 51 is also in communication with the diluent reservoir 58 from the branching portion 53a via a reagent measurement channel 56, a branching portion 53b, and a reagent introduction channel 52b. At an end portion of a channel that is branched at the branching portion 53b, the drain 57 is formed. Further, at an end portion of a channel that is branched at an end portion of the capillary channel for sample analysis 5x at the diluent reservoir 58 side, a drain 59 is formed. Furthermore, between the electrode arrangement portion 61 and the drain 63, a flow amount measurement channel 62 is formed.

In the electrophoresis chip 200 of this embodiment, the capillary channel for sample analysis 5x is filled with the electrophoresis running buffer, and the electrophoresis running buffer is measured and introduced into the diluent reservoir 58 in the following manner. First, the sample introduction portion 41, the drains 45, 55, 57, and 59 are closed, an air is sucked with a pressure reduction pump (not shown) or the like that is connected to the drain 63. Thereby, the reagent introduction channels 52a and 52b, the reagent measurement channel 56, the diluent reservoir 58, the capillary channel for sample analysis 5x, the electrode arrangement portion 61, and the flow amount measurement channel 62 are filled with an electrophoresis running buffer which is sealed in the reagent reservoir 51. Subsequently, the reagent reservoir 51 is closed, the drain 59 is opened, and an air is sucked with a pressure reduction pump (not shown) or the like that is connected to the drain 57. Thereby, an electrophoresis running buffer in the reagent introduction channel 52b and the diluent reservoir 58 is removed. Further, the drain 57 is closed, the drain 55 is opened, and an air is sucked with a pressure reduction pump (not shown) or the like that is connected to the drain 59. Thereby, an electrophoresis running buffer corresponding to the volume of the reagent measurement channel 56 can be measured and introduced into the diluent reservoir 58. Further, the sample and the electrophoresis running buffer can be mixed by introducing the sample into the diluent reservoir 58 as described above and rotating the stirring bar (not shown) using a magnetic stirrer (not shown).

In the electrophoresis chip 200 of this embodiment, the material of the upper substrate 3a is not particularly limited as long as it does not adversely affect the measurement of the absorbance. For example, the upper substrate 3a formed of the aforementioned materials can be used.

In the electrophoresis chip 200 of this embodiment, the length and the width of the upper substrate 3a are, for example, in the range of 10 mm to 200 mm, and preferably in the range of 20 mm to 100 mm. Further, the thickness of the upper substrate 3a is, for example, in the range of 0.1 mm to 10 mm, and preferably in the range of 1 mm to 5 mm.

In the electrophoresis chip 200 of this embodiment, for example, the lower substrate 3b, which is formed of acrylic resin, the material similar to that of the upper substrate 3a, or the like, can be used. The lower substrate 3b is composed by laminating plural substrates formed of the aforementioned materials. Between the plural substrates, wiring patterns made of copper foil or the like are formed.

The length and the width of the lower substrate 3b are the same as that of the upper substrate 3a. The thickness of the lower substrate 3b is, for example, in the range of 0.1 mm to 10 mm.

In the electrophoresis chip 200 of this embodiment, with respect to the diameter and the depth of the sample introduction portion 41, for example, the diameter is in the range of 0.1 mm to 10 mm and the depth is in the range of 0.1 mm to 10 mm, and preferably, the diameter is in the range of 1 mm to 5 mm and the depth is in the range of 1 mm to 5 mm.

In the electrophoresis chip 200 of this embodiment, with respect to the diameter and the depth of the reagent reservoir 51, for example, the diameter is in the range of 0.5 mm to 50 mm and the depth is in the range of 0.1 mm to 10 mm, and preferably, the diameter is in the range of 1 mm to 20 mm and the depth is in the range of 1 mm to 5 mm. Further, in the electrophoresis chip 200 of this embodiment, with respect to the diameter and the depth of the diluent reservoir 58, for example, the diameter is in the range of 0.5 mm to 50 mm and the depth is in the range of 0.1 mm to 10 mm, and preferably, the diameter is in the range of 1 mm to 10 mm and the depth is in the range of 1 mm to 5 mm.

In the electrophoresis chip 200 of this embodiment, with respect to the diameter and the depth of the drains 45, 55, 57, 59, and 63, for example, the diameter is in the range of 0.1 mm to 10 mm and the depth is in the range of 0.1 mm to 10 mm respectively, and preferably, the diameter is in the range of 1 mm to 5 mm and the depth is in the range of 1 mm to 5 mm respectively.

In the electrophoresis chip 200 of this embodiment, the form of the sample introduction portion 41, the reagent reservoir 51, the diluent reservoir 58, and the drains 45, 55, 57, 59, and 63 is cylindrical. However, the present invention is not limited thereto. In the present invention, the sample introduction portion 41, the reagent reservoir 51, the diluent reservoir 58, and the drains 45, 55, 57, 59, and 63 may be in arbitrary form and examples thereof include a quadrangular prism, a quadrangular pyramid, a cone, a combination thereof, and the like, besides a cylinder. The form of the sample introduction portion 41, the reagent reservoir 51, the diluent reservoir 58, and the drains 45, 55, 57, 59, and 63 may all be the same or may each be different.

In the electrophoresis chip 200 of this embodiment, the width and the depth of the capillary channel for sample analysis 5x are the same as that of the electrophoresis chip 2 shown in FIG. 1. Further, the distance from the electrophoresis starting point 80 to the detecting point 90 is the same as that of the electrophoresis chip 2 shown in FIG. 1.

In the electrophoresis chip 200 of this embodiment, with respect to the width and the depth of the reagent measurement channel 56 at the maximum portion of a cross-sectional area, for example, the width is in the range of 0.1 mm to 10 mm and the depth is in the range of 0.1 mm to 10 mm.

In the electrophoresis chip 200 of this embodiment, with respect to the width and the depth of the orifice 47, for example, the width is in the range of 1 $\mu$m to 200 $\mu$m and the depth is in the range of 1 $\mu$m to 200 $\mu$m, and preferably, the width is in the range of 10 $\mu$m to 100 $\mu$m and the depth is in the range of 10 $\mu$m to 100 $\mu$m.

In the electrophoresis chip 200 of this embodiment, with respect to the width and the depth of capillary channels except for the capillary channel for sample analysis 5x, the reagent measurement channel 56, and the orifice 47, for example, the width is in the range of 10 $\mu$m to 1000 $\mu$m and the depth is in the range of 10 $\mu$m to 1000 $\mu$m, and preferably, the width is in the range of 50 $\mu$m to 500 $\mu$m and the depth is in the range of 50 $\mu$m to 500 $\mu$m.

In the electrophoresis chip 200 of this embodiment, the maximum thickness of the whole electrophoresis chip is a sum of the thickness of the upper substrate 3a and the lower substrate 3b. The thickness of the whole electrophoresis chip is as described above.

The method of producing the electrophoresis chip 200 of this embodiment is not particularly limited and conventionally known methods can suitably be used, for example.

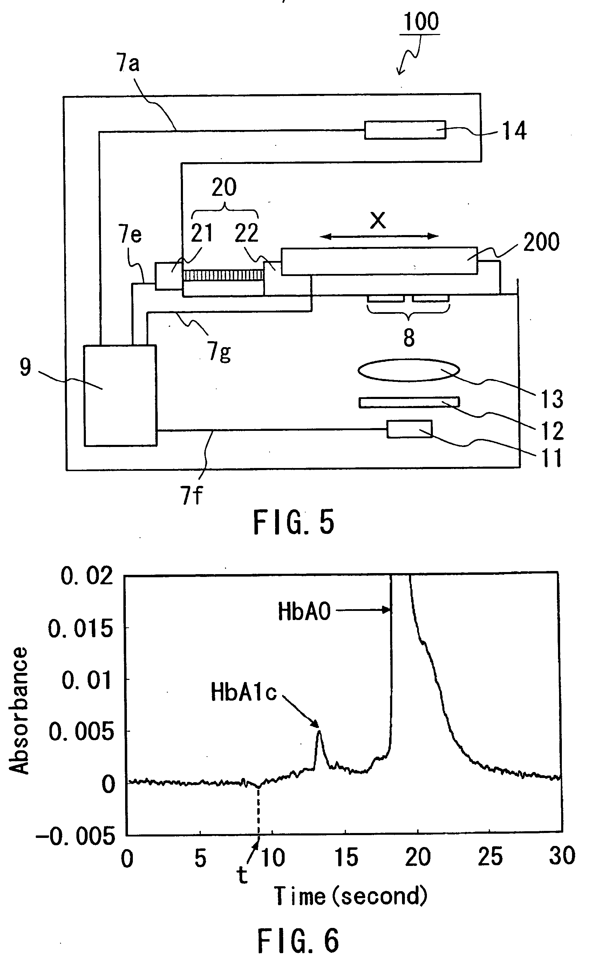

A capillary electrophoresis analysis apparatus of this embodiment is shown in FIG. 5. In FIG. 5, the portions that are identical to those in FIG. 2 are given the same numbers and symbols. As shown in FIG. 5, the capillary electrophoresis analysis apparatus 100 has the same configuration as the electrophoresis analysis apparatus 1 shown in FIG. 2 except that an electrophoresis chip is the electrophoresis chip shown in FIG. 4 instead of the electrophoresis chip shown in FIG. 1, the quantitative dispensing unit 30, the diluent 31, and the electric wires 7b to 7d are not provided as well as the electrophoresis running buffer is provided in the electrophoresis chip 200 and the connecting portion (not shown) of the connector 70 and an electric wire 7g are provided. Although it is not shown in FIG. 5, the electrophoresis chip 200 is attached to the stage 22 via the connector 70 and disposed in the capillary electrophoresis analysis apparatus 100. Further, the connector 70 is connected to the control unit 9 via the electric wire 7g. The control unit 9 controls power supply or the like to the connector 70.

Next, a method of analyzing protein in blood using the capillary electrophoresis analysis apparatus 100 of this embodiment is explained.

First, the electrophoresis chip 200 is attached to the capillary electrophoresis analysis apparatus 100 via the connector 70. Next, as described above, the capillary channel for sample analysis 5x is filled with the electrophoresis running buffer. Further, the electrophoresis running buffer is measured and introduced into the diluent reservoir 58. Then, in the same manner as described above, a human whole blood is introduced from the sample introduction portion 41 as the sample, and a human whole blood corresponding to the volume of the sample measurement channel 46 is measured and introduced into the diluent reservoir 58. The sample and the electrophoresis running buffer thus introduced are mixed in the diluent reservoir 58 and stirred by rotating the stirring bar (not shown) by a magnetic stirrer (not shown).

Next, the voltage is applied to the electrodes 6a and 6b, thereby creating a potential difference between both ends of the capillary channel for sample analysis 5x. The sample is thereby moved from the electrophoresis starting point 80 to the electrodes 6b side. The voltage application is performed by supplying power from the connector 70 to the electrodes 6a and 6b via the electric wire 7g in the wiring pattern. The voltage is not particularly limited, however is, for example, in the range of 0.5 to 20kV. As described above, the electric field for separation of the capillary channel for sample analysis 5x due to the voltage application can be set suitably according to the distance from the electrophoresis starting point to the detecting point and the width and the depth of the capillary channel. However, the electric field for separation of the capillary channel for sample analysis 5x is, for example, in the same range as Embodiment 1.

Next, in the same manner as in Embodiment 1, the light is dispersed and collected, and then the detecting point 90 is irradiated with the light (wave length of 415 nm), from which a stray light is further removed. Then, the transmitted light at the detecting point 90 is detected using the detection unit 14 and the absorbance of the protein in the sample that is subjected to the electrophoresis is measured. Electropherogram is made that indicates relationship between degree of the obtained absorbance and analysis time.

[Examples]

Next, analysis examples of glycated hemoglobin (HbA1c) using the capillary electrophoresis analysis apparatus of the present invention shown in FIG. 2 are explained.

[Example 1]

First, an electrophoresis chip shown in FIG. 1 was produced. The electrophoresis chip 2 of this Example was made of polymethylmethacrylate (PMMA). The length (dimension in the direction along the capillary channel for sample analysis 5x) of the chip was 70 mm and the width thereof was 30 mm. In the electrophoresis chip 2 of this Example, the width of the capillary channel for sample analysis 5x was 40 $\mu$m and the depth thereof was 40 $\mu$m. Further, the distance from the electrophoresis starting point 80 to the detecting point 90 was 1.5 cm.

Then, the electrophoresis chip 2 of this Example was attached to the stage 22 and the capillary electrophoresis analysis apparatus 1 shown in FIG. 2 was configured. Further, the electrophoresis running buffer 32 was prepared by adding chondroitin sulfate C in a proportion of 0.8 wt% to a solution, in which 30 mmol/L of sodium thiocyanate was added to 50 mmo/L of fumaric acid, that had been adjusted to pH 4.8 by addition of arginine. Using the electrophoresis running buffer 32, hemoglobin (manufactured by BML INC.) was diluted to a concentration of 10g/L, and thereby a sample was prepared. In this state, in the sample, a hemoglobin A1c concentration was about 5 wt% and a hemoglobin concentration thereof was corresponding to a hemoglobin concentration of a human blood that is diluted by 15 fold.

Then, the electrophoresis running buffer 32 was injected into the liquid reservoir 4a of the electrophoresis chip 2. Further, the capillary channel for sample analysis 5x was filled with the electrophoresis running buffer 32 by sucking air with a pressure reduction pump that was connected to the liquid reservoir 4b. Subsequently, the sample was introduced into the liquid reservoir 4a. Then, an electric field for separation of 450 V/cm was applied to the electrodes 6a and 6b, thereby creating a potential difference between both ends of the capillary channel for sample analysis 5x. Thereby, the sample was moved from the liquid reservoir 4a to the liquid reservoir 4b side. At this time, an absorbance (with a measurement wave length of 415 nm) at the detecting point 90 of the capillary channel for sample analysis 5x was measured using the detection unit 14. Then, electropherogram was made that indicated relationship between the absorbance and time elapsed from the start of the voltage application, i.e., analysis time (sec). Further, in the electropherogram, an electroosmotic flow was calculated using the following Formula (1) with a decreasing point of absorbance appeared before detection of hemoglobin being considered as t (sec) and the distance from the electrophoresis starting point to the detecting point being considered as d (cm).

$$\text{Electroosmotic flow (ch/min)} = d / (t / 60) \qquad (1)$$

[Example 2]

In this Example, in the same manner as in Example 1, the electrophoresis chip 2 was produced and the capillary electrophoresis analysis apparatus 1 was configured. Then, using the capillary electrophoresis analysis apparatus 1, analysis of stable hemoglobin A1c and unstable hemoglobin A1c was performed. In this Example, the analysis was performed in the same manner as in Example 1 except that 100 g/L of hemoglobin, to which 500 mg/dL of glucose was added instead of 10 g/L hemoglobin described above, that was incubated at 37°C for 3 hours and diluted by 10 fold was used as the sample.

[Example 3]

In this Example, in the same manner as in Example 1, the electrophoresis chip 2 was produced and the capillary electrophoresis analysis apparatus 1 was configured. Then, using the capillary electrophoresis analysis apparatus 1, analysis of hemoglobin A1c was performed. In this Example, the analysis was performed in the same manner as in Example 1 except that the length from the electrophoresis starting point 80 to the detecting point 90 was 1.0 cm instead of 1.5 cm described above.

[Example 4]

In this Example, in the same manner as in Example 1, the electrophoresis chip 2 was produced and the capillary electrophoresis analysis apparatus 1 was configured. Then, using the capillary electrophoresis analysis apparatus 1, analysis of hemoglobin A1c was performed. In this Example, the analysis was performed in the same manner as in Example 1 except that the length from the electrophoresis starting point 80 to the detecting point 90 was 2.0 cm instead of 1.5 cm described above.

[Example 5]

In this Example, in the same manner as in Example 1, the electrophoresis chip 2 was produced and the capillary electrophoresis analysis apparatus 1 was configured. Then, using the capillary electrophoresis analysis apparatus 1, analysis of hemoglobin A1c was performed. In this Example, the analysis was performed in the same manner as in Example 1 except that the length from the electrophoresis starting point 80 to the detecting point 90 was 0.5 cm instead of 1.5 cm described above, and an electric field for separation applied to the electrodes 6a and 6b was 250 V/cm instead of 450 V/cm described above.

[Comparative Example 1]

In this Example, in the same manner as in Example 1, the electrophoresis chip 2 was produced and the capillary electrophoresis analysis apparatus 1 was configured. Then, using the capillary electrophoresis analysis apparatus 1, analysis of hemoglobin A1c was performed. In this Example, the analysis was performed in the same manner as in Example 1 except that the hemoglobin concentration of the sample was 5 g/L (hemoglobin A1c concentration of about 11%) instead of 10 g/L, the length from the electrophoresis starting point 80 to the detecting point 90 was 2.0 cm instead of 1.5 cm, and an electric field for separation applied to the electrodes 6a and 6b was 250 V/cm instead of 450 V/cm. The hemoglobin concentration of the sample was 5g/L and was corresponding to the hemoglobin concentration of a human blood that is diluted by 30 fold.

[Comparative Example 2]

In this Example, the electrophoresis chip 2 was produced and the capillary electrophoresis analysis apparatus 1 was configured in the same manner as in Example 1 except that a separated member of capillary tube (inner diameter of 40 $\mu$m) that was embedded in a groove formed on the lower substrate 3b was used as the capillary channel for sample analysis 5x. The material of the capillary tube was fused silica. Then, using the capillary electrophoresis analysis apparatus 1, analysis of hemoglobin A1c was performed. In this Example, the analysis was performed in the same manner as in Comparative Example 1 except that the aforementioned electrophoresis chip 2 was used.

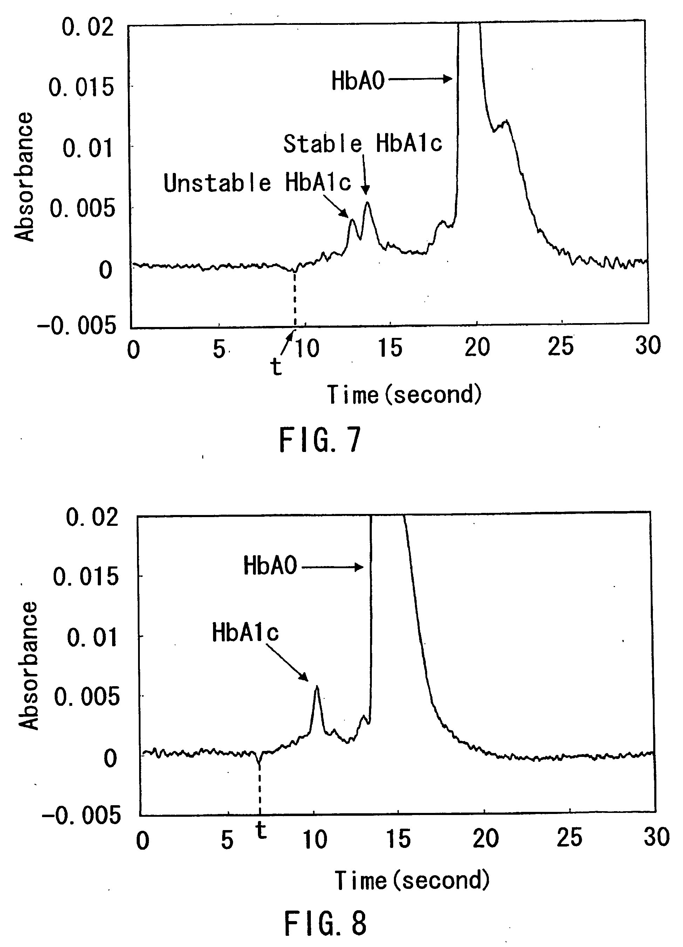

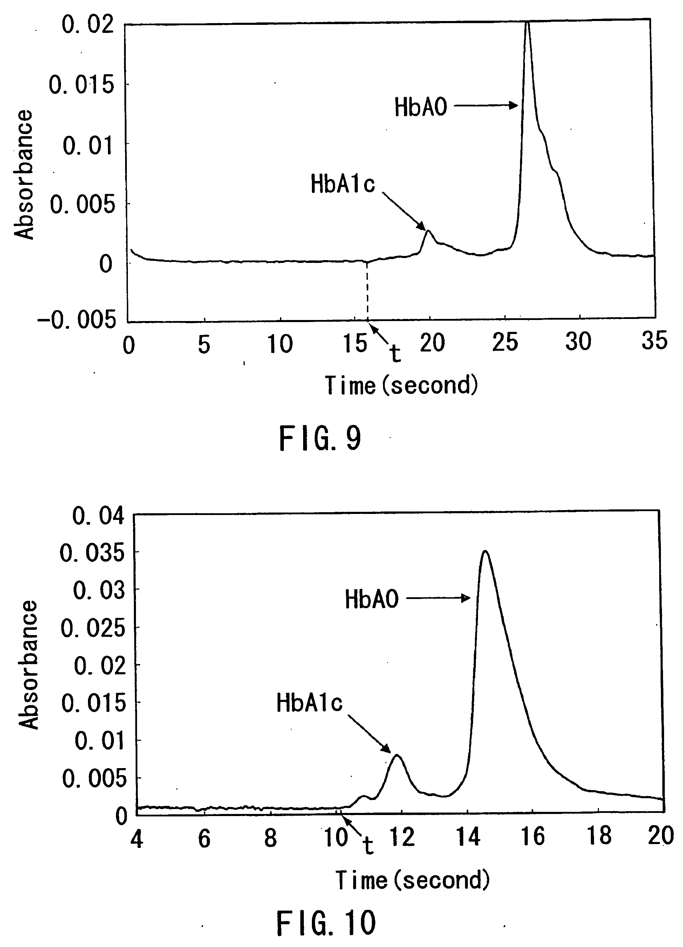

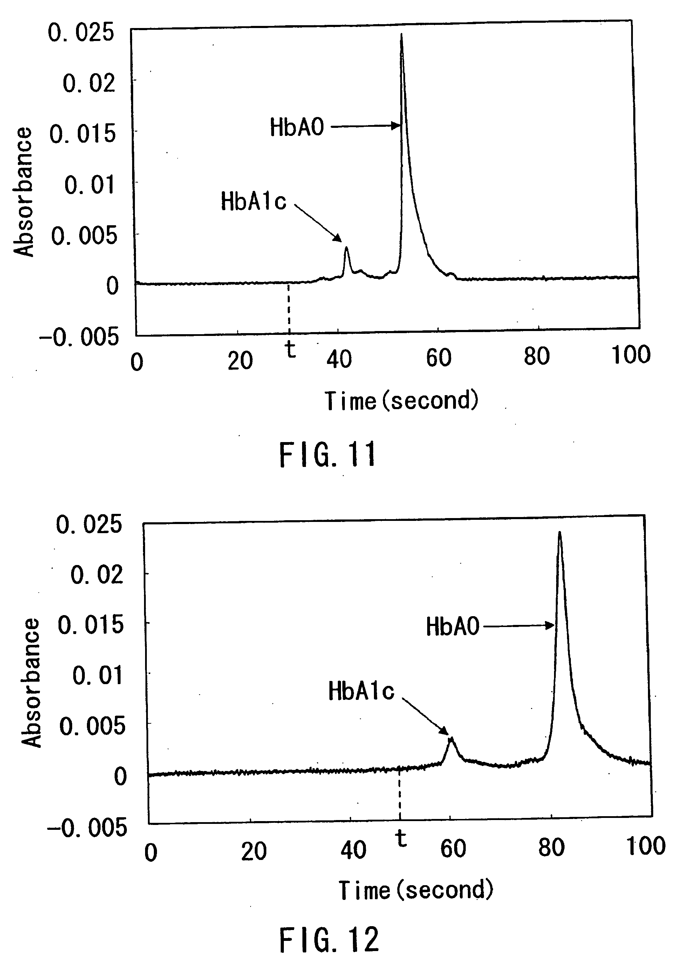

Detection results of Examples 1 to 5 and Comparative Examples 1 and 2 are shown in graphs of FIGs. 6 to 12. FIG. 6 shows the result of Example 1, FIG. 7 shows the result of Example 2, FIG. 8 shows the result of Example 3, FIG. 9 shows the result of Example 4, FIG. 10 shows the result of Example 5, FIG. 11 shows the result of Comparative Example 1, and FIG. 12 shows the result of Comparative Example 2. Further, in graphs of FIGs. 6 to 12, horizontal axes indicate the time (sec) elapsed from the start of voltage application and vertical axes indicate an absorbance at a measurement wave length of 415 nm.

Results of electroosmotic flow calculated in Examples 1 to 5 and Comparative Examples 1 and 2 are shown in the following Table 1. As shown in Table 1, with respect to the electroosmotic flow, Examples 1 and 2 were 10 cm/min, Example 3 was 8.6 cm/min, Example 4 was 7.5 cm/min, Example 5 was 3.0 cm/min, Comparative Example 1 was 4.0 cm/min, and Comparative Example 2 was 2.4 cm/min.

[Table 1]

|  | t (sec) | electroosmotic flow (cm/min) |
| --- | --- | --- |
| Example 1 | 9 | 10 |
| Example 2 | 9 | 10 |
| Example 3 | 7 | 8.6 |
| Example 4 | 16 | 7.5 |
| Example 5 | 10 | 3.0 |
| Comparative Example 1 | 30 | 4.0 |
| Comparative Example 2 | 50 | 2.4 |

As shown in graphs of FIGs. 6, and 8 to 12, in Examples 1, 3, 4, and 5, and Comparative Examples 1 and 2, hemoglobin A1c and hemoglobin A0 could separately be detected. Further, as shown in the graph in FIG. 7, in Example 2, three hemoglobin such as stable hemoglobin A1c, unstable hemoglobin A1c, and hemoglobin A0 could separately be detected. The time (analysis time of hemoglobin) from the start of voltage application to the completion of detection in each Example was about 24 seconds in Example 1, about 25 seconds in Example 2, about 18 seconds in Example 3, about 30 seconds in Example 4, and about 18 seconds in Example 5. In contrast, the time from the start of voltage application to the completion of detection was about 60 seconds in Comparative Example 1 and about 90 seconds in Comparative Example 2. In other words, although the analysis time in Examples 1 to 5 were very short such as 35 seconds or shorter, the analysis time in Comparative Examples 1 and 2 were 60 seconds or longer.

According to the present invention, the whole analysis apparatus can be downsized, operation can be simplified, running cost can be reduced, and protein in blood can be analyzed highly accurately in a short time such as 35 seconds or shorter. Particularly, the present invention is suitable for a micro total analysis systems (µTAS). The present invention is applicable to all technical fields where protein in blood is analyzed, such as laboratory tests, biochemical examinations and medical research. The intended use of the capillary electrophoresis analysis apparatus is not limited and it is applicable to a broad range of technical fields.

**Claims**

1. An analysis method for analyzing protein in blood by a capillary electrophoresis method, wherein an electrophoresis chip (2) in which a capillary channel is formed is used, the capillary channel including a capillary channel for sample analysis,
the method comprising:

introducing a sample containing the protein in blood into the capillary channel for sample analysis that is filled with an electrophoresis running buffer (32) and subjecting the sample to an electrophoresis by applying voltage to an electrode; and
measuring an absorbance of the protein in blood in the sample subjected to the electrophoresis, wherein the electrophoresis running buffer contains sulfated polysaccharide and the sulfated polysaccharide is a chondroitin sulfate, the protein in blood is considered as an analysis item, and the analysis item is hemoglobin, wherein the hemoglobin is at least one of hemoglobin A1c (HbA1c) and hemoglobin F (HbF),
**characterised in that** an analysis time of the protein in blood is 35 seconds or shorter, and any one of the following conditions (A) to (D) is satisfied:

(A) in the capillary channel for sample analysis, in a case where the cross-sectional shape is rectangular having a width and a depth of 30 μm each, when the distance from the electrophoresis starting point to the detecting point is

0.25 cm or longer and shorter than 0.75 cm, an electric field for separation is 300 V/cm or less, or

0.75 cm or longer and shorter than 1.25 cm, an electric field for separation is in a range of 300 to 600 V/cm, or

1.25 cm or longer and shorter than 1.75 cm, an electric field for separation is in a range of 400 to 650 V/cm, or

1.75 cm or longer and shorter than 2.25 cm, an electric field for separation is in a range of 450 to 700 V/cm,

(B) in the capillary channel for sample analysis, in a case where the cross-sectional shape is rectangular having a width and a depth of 40 μm each, when the distance from the electrophoresis starting point to the detecting point is

0.25 cm or longer and shorter than 0.75 cm, an electric field for separation is 250 V/cm or less, or

0.75 cm or longer and shorter than 1.25 cm, an electric field for separation is in a range of 250 to 500 V/cm, or

1.25 cm or longer and shorter than 1.75 cm, an electric field for separation is in a range of 375 to 550 V/cm, or

1.75 cm or longer and shorter than 2.25 cm, an electric field for separation is in a range of 400 to 600 V/cm,

(C) in the capillary channel for sample analysis, in a case where the cross-sectional shape is rectangular having a width and a depth of 50 μm each, when the distance from the electrophoresis starting point to the detecting point is

0.25 cm or longer and shorter than 0.75 cm, an electric field for separation is 200 V/cm or less, or

0.75 cm or longer and shorter than 1.25 cm, an electric field for separation is in a range of 200 to 450 V/cm, or

1.25 cm or longer and shorter than 1.75 cm, an electric field for separation is in a range of 300 to 500 V/cm, or

1.75 cm or longer and shorter than 2.25 cm, an electric field for separation is in a range of 350 to 550 V/cm,

(D) in the capillary channel for sample analysis, in a case where the cross-sectional shape is rectangular having a width and a depth of 60 μm each, when the distance from the electrophoresis starting point to the detecting point is

0.25 cm or longer and shorter than 0.75 cm, an electric field for separation is 150 V/cm or less, or

0.75 cm or longer and shorter than 1.25 cm, an electric field for separation is in a range of 150 to 400 V/cm, or

1.25 cm or longer and shorter than 1.75 cm, an electric field for separation is in a range of 250 to 450 V/cm, or

1.75 cm or longer and shorter than 2.25 cm, an electric field for separation is in a range of 300 to 500 V/cm.

2. The analysis method according to claim 1, wherein an inner wall surface of the capillary channel for sample analysis has an ionic functional group,
pH of the electrophoresis running buffer is in a range of 4.0 to 6.0, and
an electroosmotic flow generated at the time of voltage application is 3 cm/min or more.

3. The analysis method according to claim 1, wherein the hemoglobin A1c (HbA1c) is considered as an analysis item, and a hemoglobin A1c concentration (HbA1c concentration) is calculated as an analysis of the hemoglobin A1c (HbA1c).

4. The analysis method according to claim 1, wherein the sample is a sample prepared by subjecting the blood to a hemolysis treatment, and the hemoglobin in the sample that is prepared by subjecting the blood to the hemolysis treatment is analyzed.

5. A capillary electrophoresis analysis apparatus for analyzing protein in blood by a capillary electrophoresis method, wherein the capillary electrophoresis analysis apparatus comprises
an electrophoresis chip (2), a voltage application unit, a liquid sending unit, and an absorbance measurement unit, he electrophoresis chip comprises a substrate (3a, 3b), a plurality of liquid reservoirs (4a, 4b), and a capillary channel, the voltage application unit comprises an electrode (6a, 6b),
the plurality of liquid reservoirs are formed in the substrate,
the plurality of liquid reservoirs are in communication with one another via the capillary channel,
the capillary channel includes a capillary channel for sample analysis, and
wherein the apparatus is arranged so that, in use, the capillary channel for sample analysis can be filled with an electrophoresis running buffer using the liquid sending unit, a sample containing the protein in blood to be analyzed can be introduced into the capillary channel for sample analysis that is filled with the electrophoresis running buffer, the sample can be subjected to an electrophoresis by applying voltage to the electrode, an absorbance of the protein in blood in the sample subjected to the electrophoresis can be measured by the absorbance measurement unit, and wherein the electrophoresis running buffer contains sulfated polysaccharide and the sulfated polysaccharide is a chondroitin sulfate, the protein in blood is considered as an analysis item, and the analysis item is hemoglobin, wherein the hemoglobin is at least one of hemoglobin A1c (HbA1c) and hemoglobin F (HbF),

17

**characterised in that** an analysis time of the protein in blood is 35 seconds or shorter, and any one of the following conditions (A) to (D) is satisfied:

(A) in the capillary channel for sample analysis, in a case where the cross-sectional shape is rectangular having a width and a depth of 30 $\mu$m each, when the distance from the electrophoresis starting point to the detecting point is

0.25 cm or longer and shorter than 0.75 cm, an electric field for separation is 300 V/cm or less, or

0.75 cm or longer and shorter than 1.25 cm, an electric field for separation is in a range of 300 to 600 V/cm, or

1.25 cm or longer and shorter than 1.75 cm, an electric field for separation is in a range of 400 to 650 V/cm, or

1.75 cm or longer and shorter than 2.25 cm, an electric field for separation is in a range of 450 to 700 V/cm,

(B) in the capillary channel for sample analysis, in a case where the cross-sectional shape is rectangular having a width and a depth of 40 $\mu$m each, when the distance from the electrophoresis starting point to the detecting point is

0.25 cm or longer and shorter than 0.75 cm, an electric field for separation is 250 V/cm or less, or

0.75 cm or longer and shorter than 1.25 cm, an electric field for separation is in a range of 250 to 500 V/cm, or

1.25 cm or longer and shorter than 1.75 cm, an electric field for separation is in a range of 375 to 550 V/cm, or

1.75 cm or longer and shorter than 2.25 cm, an electric field for separation is in a range of 400 to 600 V/cm,

(C) in the capillary channel for sample analysis, in a case where the cross-sectional shape is rectangular having a width and a depth of 50 $\mu$m each, when the distance from the electrophoresis starting point to the detecting point is

0.25 cm or longer and shorter than 0.75 cm, an electric field for separation is 200 V/cm or less, or

0.75 cm or longer and shorter than 1.25 cm, an electric field for separation is in a range of 200 to 450 V/cm, or

1.25 cm or longer and shorter than 1.75 cm, an electric field for separation is in a range of 300 to 500 V/cm, or

1.75 cm or longer and shorter than 2.25 cm, an electric field for separation is in a range of 350 to 550 V/cm,

(D) in the capillary channel for sample analysis, in a case where the cross-sectional shape is rectangular having a width and a depth of 60 $\mu$m each, when the distance from the electrophoresis starting point to the detecting point is

0.25 cm or longer and shorter than 0.75 cm, an electric field for separation is 150 V/cm or less, or

0.75 cm or longer and shorter than 1.25 cm, an electric field for separation is in a range of 150 to 400 V/cm, or

1.25 cm or longer and shorter than 1.75 cm, an electric field for separation is in a range of 250 to 450 V/cm, or

1.75 cm or longer and shorter than 2.25 cm, an electric field for separation is in a range of 300 to 500 V/cm.

## Patentansprüche

1. Ein Analyseverfahren zur Analyse von Protein in Blut durch ein Kapillar-Elektrophorese-Verfahren, wobei ein Elektrophorese-Chip (2), in dem ein Kapillarkanal geformt wird, genutzt wird, wobei der Kapillarkanal einen Kapillarkanal zur Probenanalyse umfasst,
wobei das Verfahren umfasst:

eine Probe, die das Protein im Blut enthält, zur Probenanalyse in den Kapillarkanal zu geben, der mit den Elektrophoresepuffer (32) gefüllt ist, und diese Probe einer Elektrophorese zu unterziehen, indem eine Spannung an die Elektrode angelegt wird; und
die Absorption des Proteins im Blut in der Probe, für die die Elektrophorese durchgeführt wird, messen, wobei der Elektrophoresepuffer sulfatierte Polysaccharide enthält und es sich bei dem sulfatierten Polysaccharid um ein Chondroitinsulfat handelt, wobei das Protein im Blut als Analyseposition betrachtet wird und die Analyseposition Hämoglobin ist, wobei das Hämoglobin mindestens entweder Hämoglobin A1c (HbA1c) oder Hämoglobin F (HbF) ist,
**dadurch gekennzeichnet, dass** die Analysezeit des Proteins im Blut 35 Sekunden oder weniger beträgt und eine der folgenden Bedingungen (A) bis (D) erfüllt wird:

(A) im Kapillarkanal zur Probenanalyse, im Fall, dass die Querschnittsform mit einer Breite und Tiefe von jeweils 30 $\mu$m rechteckig ist, wenn der Abstand vom Startpunkt der Elektrophorese bis zum Entdeckungspunkt 0,25 cm oder länger und kürzer als 0,75 cm ist, ist ein elektrisches Feld zur Trennung 300 V/cm oder weniger, oder

0,75 cm oder länger und kürzer als 1,25 cm ist, liegt ein elektrisches Feld zur Trennung im Bereich von 300 V/cm bis 600 V/cm, oder

1,25 cm oder länger und kürzer als 1,75 cm ist, liegt ein elektrisches Feld zur Trennung im Bereich von 400 V/cm bis 650 V/cm, oder

1,75 cm oder länger und kürzer als 2,25 cm ist, liegt ein elektrisches Feld zur Trennung im Bereich von 450 V/cm bis 700 V/cm.

(B) im Kapillarkanal zur Probenanalyse, im Fall, dass die Querschnittsform rechteckig ist, mit einer Breite und Tiefe von jeweils 40 $\mu$m, wenn der Abstand vom Startpunkt der Elektrophorese bis zum Entdeckungspunkt 0,25 cm oder länger und kürzer als 0,75 cm ist, ist ein elektrisches Feld zur Trennung 250 V/cm oder weniger, oder

0,75 cm oder länger und kürzer als 1,25 cm ist, liegt ein elektrisches Feld zur Trennung im Bereich von 250 V/cm bis 500 V/cm, oder

1,25 cm oder länger und kürzer als 1,75 cm ist, liegt ein elektrisches Feld zur Trennung im Bereich von 375 V/cm bis 550 V/cm, oder

1,75 cm oder länger und kürzer als 2,25 cm ist, liegt ein elektrisches Feld zur Trennung im Bereich von 400 V/cm bis 600 V/cm.

(C) im Kapillarkanal zur Probenanalyse, im Fall, dass die Querschnittsform mit einer Breite und Tiefe von jeweils 50 $\mu$m rechteckig ist, wenn der Abstand vom Startpunkt der Elektrophorese bis zum Entdeckungspunkt 0,25 cm oder länger und kürzer als 0,75 cm ist, ist ein elektrisches Feld zur Trennung 200 V/cm oder weniger, oder

0,75 cm oder länger und kürzer als 1,25 cm ist, liegt ein elektrisches Feld zur Trennung im Bereich von 200 V/cm bis 450 V/cm, oder

1,25 cm oder länger und kürzer als 1,75 cm ist, liegt ein elektrisches Feld zur Trennung im Bereich von 300 V/cm bis 500 V/cm, oder

1,75 cm oder länger und kürzer als 2,25 cm ist, liegt ein elektrisches Feld zur Trennung im Bereich von 350 V/cm bis 550 V/cm.

(D) im Kapillarkanal zur Probenanalyse, im Fall, dass die Querschnittsform rechteckig ist, mit einer Breite und Tiefe von jeweils 60 $\mu$m, wenn der Abstand vom Startpunkt der Elektrophorese bis zum Entdeckungspunkt 0,25 cm oder länger und kürzer als 0,75 cm ist, ist ein elektrisches Feld zur Trennung 150 V/cm oder weniger, oder

0,75 cm oder länger und kürzer als 1,25 cm ist, liegt ein elektrisches Feld zur Trennung im Bereich von 150 V/cm bis 400 V/cm, oder

1,25 cm oder länger und kürzer als 1,75 cm ist, liegt ein elektrisches Feld zur Trennung im Bereich von 250 V/cm bis 450 V/cm, oder

1,75 cm oder länger und kürzer als 2,25 cm ist, liegt ein elektrisches Feld zur Trennung im Bereich von 300 V/cm bis 500 V/cm.

2. Das Analyseverfahren gemäß Anspruch 1, wobei eine Innenwandfläche des Kapillarkanals zur Probenanalyse eine ionische Funktionsgruppe hat, der pH- Wert des Elektrophoresepuffers im Bereich zwischen 4,0 und 6,0 liegt und ein in der Zeit der angelegten Spannung erzeugter elektroosmotischer Fluss 3 cm/min oder mehr beträgt.

3. Das Analyseverfahren gemäß Anspruch 1, wobei das Hämoglobin (HbA1c) als Analyseposition angesehen wird, und eine Hämoglobin-A1c-Konzentration (HbA1c-Konzentration) als Analyse des Hämoglobins A1 c (HbA1 c) berechnet wird.

4. Das Analyseverfahren gemäß Anspruch 1, wobei die Probe eine Probe ist, die vorbereitet wurde, indem das Blut einer Hämolyse-Behandlung unterzogen wurde und das Hämoglobin in der Probe, die vorbereitet wurde, indem das Blut einer Hämolyse-Behandlung unterzogen wurde, analysiert wird.

5. Ein Kapillar-Elektrophorese-Analysegerät zur Analyse von Protein im Blut durch ein Kapillar-Elektrophorese-Verfahren, wobei das Kapillar-Elektrophorese-Analysegerät umfasst:

einen Elektrophorese-Chip (2) eine Einheit zum Anlegen der Spannung, eine Flüssigkeits-Sendeeinheit und eine Einheit zur Absorptionsmessung,
wobei der Elektrophorese-Chip ein Substrat (3a, 3b), mehrere Flüssigkeitsbehälter (4a, 4b) und einen Kapillarkanal umfasst,
die Einheit zum Anlegen der Spannung eine Elektrode (6a, 6b) umfasst,
die verschiedenen Flüssigkeitsbehälter im Substrat geformt werden,
die verschiedenen Flüssigkeitsbehälter miteinander über den Kapillarkanal in Verbindung stehen,

der Kapillarkanal einen Kapillarkanal zur Probeanalyse umfasst, und

wobei das Gerät so angeordnet ist, dass bei Verwendung, der Kapillarkanal zur Probeanalyse mit einem Elektrophoresepuffer unter Verwendung der Flüssigkeits- Sendeeinheit gefüllt werden kann, eine Probe, die das zu analysierende Protein im Blut enthält,

in den Kapillarkanal eingeführt werden kann, der mit dem Elektrophoresepuffer gefüllt, ist, die Probe einer Elektrophorese unterzogen werden kann, indem Spannung an der Elektrode angelegt wird, die Absorption des Proteins im Blut in der Probe,

die einer Elektrophorese unterzogen wird, durch die Absorptions-Messeinheit gemessen werden kann, und wobei der Elektrophoresepuffer sulfatierte Polysaccharide enthält und es sich bei dem sulfatierten Polysaccharid um ein Chondroitinsulfat handelt, das Protein im Blut wird als Analyseposition betrachtet,

und die Analyseposition ist Hämoglobin, wobei das Hämoglobin mindestens entweder Hämoglobin A1c (HbA1c) oder Hämoglobin F (HbF) ist,

**dadurch gekennzeichnet, dass** die Analysezeit des Proteins im Blut 35 Sekunden oder weniger beträgt; und eine der folgenden Bedingungen (A) bis (D) erfüllt wird:

(A) im Kapillarkanal zur Probenanalyse, im Fall, dass die Querschnittsform mit einer Breite und Tiefe von jeweils 30 $\mu$m rechteckig ist, wenn der Abstand vom Startpunkt der Elektrophorese bis zum Entdeckungspunkt 0,25 cm oder länger und kürzer als 0,75 cm ist, ist ein elektrisches Feld zur Trennung 300 V/cm oder weniger, oder

0,75 cm oder länger und kürzer als 1,25 cm ist, liegt ein elektrisches Feld zur Trennung im Bereich von 300 V/cm bis 600 V/cm, oder

1,25 cm oder länger und kürzer als 1,75 cm ist, liegt ein elektrisches Feld zur Trennung im Bereich von 400 V/cm bis 650 V/cm, oder

1,75 cm oder länger und kürzer als 2,25 cm ist, liegt ein elektrisches Feld zur Trennung im Bereich von 450 V/cm bis 700 V/cm.

(B) im Kapillarkanal zur Probenanalyse, im Fall, dass die Querschnittsform mit einer Breite und Tiefe von jeweils 40 $\mu$m rechteckig ist, wenn der Abstand vom Startpunkt der Elektrophorese bis zum Entdeckungspunkt 0,25 cm oder länger und kürzer als 0,75 cm ist, ist ein elektrisches Feld zur Trennung 250 V/cm oder weniger, oder

0,75 cm oder länger und kürzer als 1,25 cm ist, liegt ein elektrisches Feld zur Trennung im Bereich von 250 V/cm bis 500 V/cm, oder

1,25 cm oder länger und kürzer als 1,75 cm ist, liegt ein elektrisches Feld zur Trennung im Bereich von 375 V/cm bis 550 V/cm, oder

1,75 cm oder länger und kürzer als 2,25 cm ist, liegt ein elektrisches Feld zur Trennung im Bereich von 400 V/cm bis 600 V/cm.

(C) im Kapillarkanal zur Probenanalyse, im Fall, dass die Querschnittsform mit einer Breite und Tiefe von jeweils 50 $\mu$m rechteckig ist, wenn der Abstand vom Startpunkt der Elektrophorese bis zum Entdeckungspunkt 0,25 cm oder länger und kürzer als 0,75 cm ist, ist ein elektrisches Feld zur Trennung 200 V/cm oder weniger, oder

0,75 cm oder länger und kürzer als 1,25 cm ist, liegt ein elektrisches Feld zur Trennung im Bereich von 200 V/cm bis 450 V/cm, oder

1,25 cm oder länger und kürzer als 1,75 cm ist, liegt ein elektrisches Feld zur Trennung im Bereich von 300 V/cm bis 500 V/cm, oder

1,75 cm oder länger und kürzer als 2,25 cm ist, liegt ein elektrisches Feld zur Trennung im Bereich von 350 V/cm bis 550 V/cm, oder

(D) im Kapillarkanal zur Probenanalyse, im Fall, dass die Querschnittsform mit einer Breite und Tiefe von jeweils 60 $\mu$m rechteckig ist, wenn der Abstand vom Startpunkt der Elektrophorese bis zum Entdeckungspunkt 0,25 cm oder länger und kürzer als 0,75 cm ist, ist ein elektrisches Feld zur Trennung 150 V/cm oder weniger, oder

0,75 cm oder länger und kürzer als 1,25 cm ist, liegt ein elektrisches Feld zur Trennung im Bereich von 150 V/cm bis 400 V/cm, oder

1,25 cm oder länger und kürzer als 1,75 cm ist, liegt ein elektrisches Feld zur Trennung im Bereich von 250 V/cm bis 450 V/cm, oder

1,75 cm oder länger und kürzer als 2,25 cm ist, liegt ein elektrisches Feld zur Trennung im Bereich von 300 V/cm bis 500 V/cm.

**Revendications**

1. Procédé d'analyse pour analyser une protéine dans le sang par un procédé d'électrophorèse capillaire, dans lequel on utilise une puce d'électrophorèse (2) dans laquelle un canal capillaire est formé, le canal capillaire incluant un canal capillaire pour l'analyse d'échantillon,
le procédé comprenant :

l'introduction d'un échantillon contenant la protéine dans le sang dans le canal capillaire pour l'analyse d'échantillon qui est remplie d'un tampon électrophorétique (32) et la soumission de l'échantillon à une électrophorèse en appliquant une tension à une électrode ; et
la mesure d'une absorbance de la protéine dans le sang dans l'échantillon soumis à l'électrophorèse, dans lequel le tampon électrophorétique contient du polysaccharide sulfaté et le polysaccharide sulfaté est un sulfate de chondroïtine, on considère la protéine dans le sang comme un élément d'analyse, et l'élément d'analyse est de l'hémoglobine, dans laquelle l'hémoglobine est au moins une d'hémoglobine A1c (HbA1c) et d'hémoglobine F (HbF),
**caractérisé en ce qu'**un temps d'analyse de la protéine dans le sang est de 35 secondes ou plus court, et n'importe laquelle des conditions suivantes (A) à (D) est satisfaite :

(A) dans le canal capillaire pour l'analyse d'échantillon, dans un cas où la forme en coupe transversale est rectangulaire ayant une largeur et une profondeur de 30 $\mu$m chacune, quand la distance allant du point de départ d'électrophorèse au point de détection est
0,25 cm ou plus longue et plus courte que 0,75 cm, un champ électrique pour la séparation est de 300 V/cm ou moins, ou
0,75 cm ou plus longue et plus courte que 1,25 cm, un champ électrique pour la séparation est dans une plage de 300 à 600 V/cm, ou
1,25 cm ou plus longue et plus courte que 1,75 cm, un champ électrique pour la séparation est dans une plage de 400 à 650 V/cm, ou
1,75 cm ou plus longue et plus courte que 2,25 cm, un champ électrique pour la séparation est dans une plage de 450 à 700 V/cm,
(B) dans le canal capillaire pour l'analyse d'échantillon, dans un cas où la forme en coupe transversale est rectangulaire ayant une largeur et une profondeur de 40 $\mu$m chacune, quand la distance allant du point de départ d'électrophorèse au point de détection est
0,25 cm ou plus longue et plus courte que 0,75 cm, un champ électrique pour la séparation est de 250 V/cm ou moins, ou
0,75 cm ou plus longue et plus courte que 1,25 cm, un champ électrique pour la séparation est dans une plage de 250 à 500 V/cm, ou
1,25 cm ou plus longue et plus courte que 1,75 cm, un champ électrique pour la séparation est dans une plage de 375 à 550 V/cm, ou
1,75 cm ou plus longue et plus courte que 2,25 cm, un champ électrique pour la séparation est dans une plage de 400 à 600 V/cm,
(C) dans le canal capillaire pour l'analyse d'échantillon, dans un cas où la forme en coupe transversale est rectangulaire ayant une largeur et une profondeur de 50 $\mu$m chacune, quand la distance allant du point de départ d'électrophorèse au point de détection est
0,25 cm ou plus longue et plus courte que 0,75 cm, un champ électrique pour la séparation est de 200 V/cm ou moins, ou
0,75 cm ou plus longue et plus courte que 1,25 cm, un champ électrique pour la séparation est dans une plage de 200 à 450 V/cm, ou
1,25 cm ou plus longue et plus courte que 1,75 cm, un champ électrique pour la séparation est dans une plage de 300 à 500 V/cm, ou
1,75 cm ou plus longue et plus courte que 2,25 cm, un champ électrique pour la séparation est dans une plage de 350 à 550 V/cm,
(D) dans le canal capillaire pour l'analyse d'échantillon, dans un cas où la forme en coupe transversale est rectangulaire ayant une largeur et une profondeur de 60 $\mu$m chacune, quand la distance allant du point de départ d'électrophorèse au point de détection est
0,25 cm ou plus longue et plus courte que 0,75 cm, un champ électrique pour la séparation est de 150 V/cm ou moins, ou

0,75 cm ou plus longue et plus courte que 1,25 cm, un champ électrique pour la séparation est dans une plage de 150 à 400 V/cm, ou

1,25 cm ou plus longue et plus courte que 1,75 cm, un champ électrique pour la séparation est dans une plage de 250 à 450 V/cm, ou

1,75 cm ou plus longue et plus courte que 2,25 cm, un champ électrique pour la séparation est dans une plage de 300 à 500 V/cm.

2. Procédé d'analyse selon la revendication 1, dans lequel une surface de paroi intérieure du canal capillaire pour l'analyse d'échantillon a un groupe fonctionnel ionique,
le pH du tampon électrophorétique est dans une plage de 4,0 à 6,0, et un flux électro-osmotique produit au moment de l'application de tension est de 3 cm/min ou plus.

3. Procédé d'analyse selon la revendication 1, dans lequel on considère l'hémoglobine A1c (HbA1c) comme un élément d'analyse, et une concentration d'hémoglobine A1c (concentration de HbA1c) est calculée comme une analyse de l'hémoglobine A1c (HbA1c).

4. Procédé d'analyse selon la revendication 1, dans lequel l'échantillon est un échantillon préparé en soumettant le sang à un traitement d'hémolyse, et l'hémoglobine dans l'échantillon qui est préparée en soumettant le sang au traitement d'hémolyse est analysée.

5. Appareil d'analyse par électrophorèse capillaire pour analyser une protéine dans le sang par un procédé d'électrophorèse capillaire, dans lequel l'appareil l'analyse par électrophorèse capillaire comprend
une puce d'électrophorèse (2), une unité d'application de tension, une unité d'envoi de liquide, et une unité de mesure d'absorbance,
la puce d'électrophorèse comprend un substrat (3a, 3b), une pluralité de réservoirs de liquide (4a, 4b), et un canal capillaire,
l'unité d'application de tension comprend une électrode (6a, 6b),
la pluralité de réservoirs de liquide est formée dans le substrat,
la pluralité de réservoirs de liquide est en communication entre eux via le canal capillaire,
le canal capillaire inclut un canal capillaire pour l'analyse d'échantillon, et
dans lequel l'appareil est agencé de sorte que, lors de l'utilisation, le canal capillaire pour l'analyse d'échantillon peut être rempli avec un tampon électrophorétique utilisant l'unité d'envoi de liquide, un échantillon contenant la protéine dans le sang à analyser peut être introduit dans le canal capillaire pour l'analyse d'échantillon qui est rempli du tampon électrophorétique, l'échantillon peut être soumis à une électrophorèse en appliquant une tension à l'électrode, une absorbance de la protéine dans le sang dans l'échantillon soumis à l'électrophorèse peut être mesurée par l'unité de mesure d'absorbance, et dans lequel le tampon électrophorétique contient du polysaccharide sulfaté et le polysaccharide sulfaté est un sulfate de chondroïtine, on considère la protéine dans le sang comme un élément d'analyse, et l'élément d'analyse est de l'hémoglobine, dans laquelle l'hémoglobine est au moins une d'hémoglobine A1c (HbA1c) et d'hémoglobine F (HbF),
**caractérisé en ce qu'**un temps d'analyse de la protéine dans le sang est 35 secondes ou plus court, et n'importe laquelle des conditions suivantes (A) à (D) est satisfaite :

(A) dans le canal capillaire pour l'analyse d'échantillon, dans un cas où la forme en coupe transversale est rectangulaire ayant une largeur et une profondeur de 30 $\mu$m chacune, quand la distance allant du point de départ d'électrophorèse au point de détection est
0,25 cm ou plus longue et plus courte que 0,75 cm, un champ électrique pour la séparation est de 300 V/cm ou moins, ou
0,75 cm ou plus longue et plus courte que 1,25 cm, un champ électrique pour la séparation est dans une plage de 300 à 600 V/cm, ou
1,25 cm ou plus longue et plus courte que 1,75 cm, un champ électrique pour la séparation est dans une plage de 400 à 650 V/cm, ou
1,75 cm ou plus longue et plus courte que 2,25 cm, un champ électrique pour la séparation est dans une plage de 450 à 700 V/cm,
(B) dans le canal capillaire pour l'analyse d'échantillon, dans un cas où la forme en coupe transversale est rectangulaire ayant une largeur et une profondeur de 40 $\mu$m chacune, quand la distance allant du point de départ d'électrophorèse au point de détection est
0,25 cm ou plus longue et plus courte que 0,75 cm, un champ électrique pour la séparation est 250 V/cm ou moins, ou

0,75 cm ou plus longue et plus courte que 1,25 cm, un champ électrique pour la séparation est dans une plage de 250 à 500 V/cm, ou

1,25 cm ou plus longue et plus courte que 1,75 cm, un champ électrique pour la séparation est dans une plage de 375 à 550 V/cm, ou

1,75 cm ou plus longue et plus courte que 2,25 cm, un champ électrique pour la séparation est dans une plage de 400 à 600 V/cm,

(C) dans le canal capillaire pour l'analyse d'échantillon, dans un cas où la forme en coupe transversale est rectangulaire ayant une largeur et une profondeur de 50 $\mu$m chacune, quand la distance allant du point de départ d'électrophorèse au point de détection est

0,25 cm ou plus longue et plus courte que 0,75 cm, un champ électrique pour la séparation est de 200 V/cm ou moins, ou

0,75 cm ou plus longue et plus courte que 1,25 cm, un champ électrique pour la séparation est dans une plage de 200 à 450 V/cm, ou

1,25 cm ou plus longue et plus courte que 1,75 cm, un champ électrique pour la séparation est dans une plage de 300 à 500 V/cm, ou

1,75 cm ou plus longue et plus courte que 2,25 cm, un champ électrique pour la séparation est dans une plage de 350 à 550 V/cm,

(D) dans le canal capillaire pour l'analyse d'échantillon, dans un cas où la forme en coupe transversale est rectangulaire ayant une largeur et une profondeur de 60 $\mu$m chacune, quand la distance allant du point de départ d'électrophorèse au point de détection est

0,25 cm ou plus longue et plus courte que 0,75 cm, un champ électrique pour la séparation est 150 V/cm ou moins, ou

0,75 cm ou plus longue et plus courte que 1,25 cm, un champ électrique pour la séparation est dans une plage de 150 à 400 V/cm, ou

1,25 cm ou plus longue et plus courte que 1,75 cm, un champ électrique pour la séparation est dans une plage de 250 à 450 V/cm, ou

1,75 cm ou plus longue et plus courte que 2,25 cm, un champ électrique pour la séparation est dans une plage de 300 à 500 V/cm.

FIG. 1A

FIG. 1B

FIG. 2

FIG. 3A

FIG. 3B

FIG. 3C

FIG. 4A

FIG. 4B

FIG. 5

FIG. 6

FIG. 7

FIG. 8

FIG. 9

FIG. 10

FIG. 11

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3429709 B
- WO 2008047703 A1
- US 2002092770 A
- US 5431793 A
- WO 2008029684 A
- WO 2008139867 A